(19) Europäisches Patentamt
European Patent Office
Office européen des brevets

(11) EP 4 299 074 A1

(12) **EUROPEAN PATENT APPLICATION**
published in accordance with Art. 153(4) EPC

(43) Date of publication:
**03.01.2024 Bulletin 2024/01**

(21) Application number: **22759797.8**

(22) Date of filing: **25.02.2022**

(51) International Patent Classification (IPC):
***A61K 45/00** (2006.01)* ***A61P 25/00** (2006.01)*
***A61P 25/18** (2006.01)* ***A61P 25/22** (2006.01)*
***A61P 25/24** (2006.01)* ***A61P 43/00** (2006.01)*
***A61K 31/439** (2006.01)* ***A61K 31/444** (2006.01)*
***A61K 31/4725** (2006.01)* ***A61K 31/497** (2006.01)*
***A61K 31/501** (2006.01)* ***A61K 31/506** (2006.01)*

(52) Cooperative Patent Classification (CPC):
**A61K 31/439; A61K 31/444; A61K 31/4725; A61K 31/497; A61K 31/501; A61K 31/506; A61K 45/00; A61P 25/00; A61P 25/18; A61P 25/22; A61P 25/24; A61P 43/00**

(86) International application number:
**PCT/JP2022/007866**

(87) International publication number:
**WO 2022/181763 (01.09.2022 Gazette 2022/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.02.2021 JP 2021030974**
**15.07.2021 JP 2021117338**

(71) Applicants:
- **TOKYO UNIVERSITY OF SCIENCE FOUNDATION**
**Tokyo 162-8601 (JP)**
- **Nippon Chemiphar Co., Ltd.**
**Tokyo 101-0032 (JP)**

(72) Inventors:
- **SAITOH Akiyoshi**
**Tokyo 162-8601 (JP)**
- **YAMADA Daisuke**
**Tokyo 162-8601 (JP)**
- **NAKATA Eriko**
**Misato-shi, Saitama 341-0005 (JP)**

(74) Representative: **Godemeyer Blum Lenze Patentanwälte Partnerschaft mbB - werkpatent**
**An den Gärten 7**
**51491 Overath (DE)**

# (54) PHARMACEUTICAL COMPOSITION FOR TREATMENT OR PREVENTION OF STRESS-RELATED DISORDERS

(57) The present invention provides a pharmaceutical composition for the treatment or prevention of stress-related disorders, stress-induced anxiety disorders, or stress-related disorders or anxiety disorders associated with depression, the composition containing a selective δ-opioid receptor agonist as an active component, wherein the selective δ-opioid receptor agonist preferably also has a μ-opioid receptor antagonist action and a κ-opioid receptor antagonist action.

FIG. 1

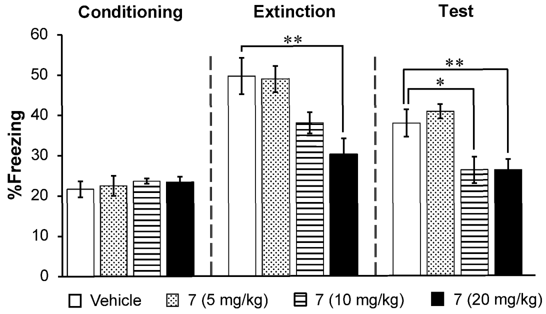


*P < 0.05, **P < 0.01 (two-way ANOVA, post hoc Bonferroni's test)

EP 4 299 074 A1

## Description

[Technical Field]

The present invention relates to a pharmaceutical composition for the treatment or prevention of stress-related disorders, stress-induced anxiety disorders, or stress-related disorders or anxiety disorders associated with depression, the pharmaceutical composition containing a selective δ-opioid receptor agonist as an active component.

Priority is claimed on Japanese Patent Application No. 2021-030974, filed February 26, 2021, and Japanese Patent Application No. 2021-117338, filed July 15, 2021, the contents of which are incorporated herein by reference.

[Background Art]

Opioids exhibit their effects by binding to opioid receptors, and there are three subtypes of opioid receptors, namely μ, δ and κ receptors. It is known that agonists of all three of these subtypes, namely μ, δ and κ receptors, have analgesic effects.

Among these agonists, it is anticipated that agonists that selectively activate the δ-opioid receptor will either not display or scarcely display the side effects that are induced via activation of the μ-opioid receptor or x-opioid receptor.

Various compounds have already been reported as δ-opioid receptor agonists, and the analgesic effects, antidepressant effects and anxiolytic effects of these compounds have been verified (see Patent documents 1 to 6, and Non-Patent Documents 1 to 3).

However, the formation of fear memories is a defense reaction aimed at avoiding danger or threats, and is an instinctive behavior seen across all animals. Once acquired, fear memories are retained for long periods, but if new learning occurs that indicates fear need no longer be felt, then extinction of these memories can occur. For example, post-traumatic stress disorder (sometimes abbreviated as PTSD) is a condition in which fear memories formed as a result of fear experiences are not extinguished even when that fear need no longer be felt, and it is thought that anomalies in fear memory control are related to the appearance and state of this disorder. The main symptoms of PTSD include repeated re-experience symptoms such as flashbacks or nightmares, mental numbness symptoms caused by avoidance of memory cue or a deterioration in mental activity, or hyperarousal symptoms such as irritability or insomnia. Conventionally, in the pharmaceutical treatment of PTSD, antidepressant drugs such as selective serotonin reuptake inhibitors (sometimes abbreviated as SSRI) or serotonin-noradrenaline reuptake inhibitors (sometimes abbreviated as SNRI) have been the drugs of first choice, with atypical antipsychotic drugs or benzodiazepine-based drugs being the drugs of second choice, but all these types of drugs represent symptomatic treatment, and drugs having selectivity for fear neural circuits are yet to be developed.

On the other hand, the extinction-facilitating effects of δ-opioid receptor agonists on anxiety or fear memories have already been reported for SNC80 and KNT-127 (see Non-Patent Documents 4 and 5). Contextual fear conditioning experiments were used as an animal model, and although the above compounds exhibited an anxiolytic effect through the extinction of anxiety or fear memories, only KNT-127 was found to provide an extinction learning-facilitating effect for anxiety or fear memories. Further, in the case of κ-opioid receptors (sometimes abbreviated as KOR), it has been suggested that the endogenous peptide dynorphin is released in states of high stress, with KOR activation contributing to the formation and extinction of aversive memories (see Non-Patent Document 6).

However, in the treatment of PTSD and the like, although cognitive behavior therapy is also used, the therapeutic effects are unsatisfactory and the prognosis is poor, meaning a new treatment method is needed.

[Citation List]

[Patent Documents]

[Patent Document 1]
JP2006-522775
[Patent Document 2]
WO2001/046192
[Patent Document 3]
WO2008/001859
[Patent Document 4]
WO2013/035833
[Patent Document 5]

WO2014/021273
[Patent Document 6]
WO2014/136305

[Non-Patent Documents]

[Non-Patent Document 1]
Tetrahedron, 2011, 67, 6682 to 6668
[Non-Patent Document 2]
European Journal of Pharmacology 276 (1995) 131 to 135
[Non-Patent Document 3]
European Journal of Pharmacology 322 (1997) 27 to 30
[Non-Patent Document 4]
Neuropharmacology, 160 (2019) 107792
[Non-Patent Document 5]
Journal of Pharmacological Sciences 139(3) (2019) 174 to 179
[Non-Patent Document 6]
Journal of Neuroscience 32(27) (2012) 9335 to 9343

[Summary of Invention]

[Technical Problem]

An object of the present invention is to provide a pharmaceutical composition that is useful for the treatment or prevention of stress-related disorders, stress-induced anxiety disorders, or stress-related disorders or anxiety disorders associated with depression.

[Solution to Problem]

As a result of intensive investigation aimed at achieving the object described above, the inventors of the present invention discovered that a pharmaceutical composition containing a selective δ-opioid receptor agonist as an active component, wherein the selective δ-opioid receptor agonist preferably also has a μ-opioid receptor antagonist action and a κ-opioid receptor antagonist action, was useful for the treatment or prevention of stress-related disorders, stress-induced anxiety disorders, or stress-related disorders or anxiety disorders associated with depression, and they were thus able to complete the present invention.
In other words, the present invention includes the following aspects.
[1] A pharmaceutical composition for the treatment or prevention of stress-related disorders, stress-induced anxiety disorders, or stress-related disorders or anxiety disorders associated with depression, the composition containing a selective δ-opioid receptor agonist as an active component;
[2] The pharmaceutical composition for treatment or prevention according to [1], wherein the selective δ-opioid receptor agonist also has a κ-opioid receptor antagonist action;
[3] The pharmaceutical composition for treatment or prevention according to [1] or [2], wherein the selective δ-opioid receptor agonist also has a μ-opioid receptor antagonist action and a κ-opioid receptor antagonist action;
[4] The pharmaceutical composition for treatment or prevention according to any one of [1] to [3], wherein the stress-related disorder is an acute stress disorder, post-traumatic stress disorder, or adjustment disorder;
[5] The pharmaceutical composition for treatment or prevention according to any one of [1] to [4], wherein the stress-related disorder is an acute stress disorder or post-traumatic stress disorder;
[6] The pharmaceutical composition for treatment or prevention according to [4] or [5] which functions as a pharmaceutical composition for the treatment or prevention of post-traumatic stress disorder, and has an inhibitory effect on intrusion symptoms related to the traumatic event, an inhibitory effect on persistent avoidance of stimuli associated with the traumatic event, or an inhibitory effect on negative alterations in cognitions and mood associated with the traumatic event, that begin following the traumatic event;
[7] The pharmaceutical composition for treatment or prevention according to [6], wherein the intrusion symptoms related to the traumatic event that begin following the traumatic event include at least one symptom among (1) to

(5) described below:

(1) recurrent, involuntary and intrusive distressing memories of the traumatic event;
(2) recurrent distressing dreams in which the dream content and/or emotions are related to the traumatic event;
(3) dissociative symptoms causing feelings or actions as if the traumatic event were recurring;
(4) intense or prolonged psychological distress upon exposure to internal or external cues that symbolize or resemble an aspect of the traumatic event; and
(5) marked physiological reactions to internal or external cues that symbolize or resemble an aspect of the traumatic event;

[8] The pharmaceutical composition for treatment or prevention according to any one of [1] to [7], wherein the composition suppresses flashbacks;
[9] The pharmaceutical composition for treatment or prevention according to any one of [1] to [8], wherein the composition suppresses avoidance of events or matters related to a trauma;
[10] The pharmaceutical composition for treatment or prevention according to any one of [1] to [9], wherein the composition has a facilitation effect on the extinction of memories of a traumatic event, and/or an inhibitory effect on the reconsolidation of memories of a traumatic event;
[11] The pharmaceutical composition for treatment or prevention according to [10], wherein the facilitation effect on the extinction of memories of a traumatic event is a facilitation effect on extinction learning of memories of a traumatic event;
[12] The pharmaceutical composition for treatment or prevention according to any one of [1] to [11], wherein the composition palliates fear from fear memories;
[13] The selective opioid delta receptor agonist is a compound represented by the following general formula (I):

[Formula 1]

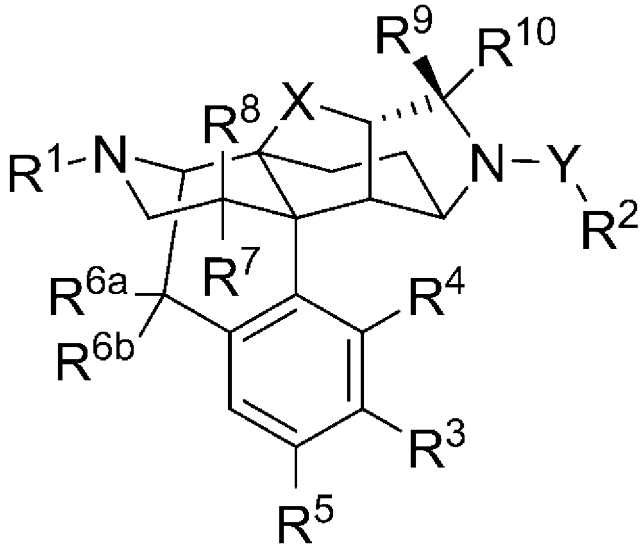

(I)

(wherein $R^1$ represents hydrogen; $C_{1-10}$ alkyl; $C_{6-10}$ aryl; $C_{2-6}$ alkenyl; cycloalkylalkyl where the cycloalkyl moiety has 3 to 6 carbon atoms, and the alkylene moiety has 1 to 5 carbon atoms; aralkyl where the aryl moiety has 6 to 10 carbon atoms, and the alkylene moiety has 1 to 5 carbon atoms; $C_{3-6}$ cycloalkyl; or heteroarylalkyl where the heteroaryl moiety contains 1 to 4 heteroatoms selected from N, O and S as ring-constituting atoms, and the alkylene moiety has 1 to 5 carbon atoms,

$R^2$ represents heterocyclic ring containing 1 to 4 heteroatoms selected from N, O and S and at least one carbon atom as ring-constituting atoms, containing at least one set of adjacent ring-constituting atoms

bound by a double bond, and further substituted with at least one oxo group; or pyridine 1-oxide,

$R^2$ binds to Y via a carbon atom as a ring-constituting atom of $R^2$,

R3, $R^4$, and $R^5$, which are the same or different, represent hydrogen; hydroxy; halogen; cyano; carbamoyl; $C_{1-6}$ alkoxy; $C_{6-10}$ aryloxy; $C_{1-6}$ alkanoyloxy; nitro; amino; $C_{1-8}$ alkylamino; $C_{6-10}$ arylamino; or acylamino where the acyl moiety has 2 to 6 carbon atoms,

$R^{6a}$ and $R^{6b}$, which are the same or different, represent hydrogen; fluorine; or hydroxy, or $R^{6a}$ and $R^{6b}$ combine together to represent =O,

$R^7$ and $R^8$, which are the same or different, represent hydrogen; fluorine; or hydroxy,

$R^9$ and $R^{10}$, which are the same or different, represent hydrogen; $C_{1-6}$ alkyl; $C_{6-10}$ aryl; heteroaryl containing 1 to 4 heteroatoms selected from N, O and S as ring-constituting atoms; aralkyl where the aryl moiety has 6 to 10 carbon atoms, and the alkylene moiety has 1 to 5 carbon atoms; heteroarylalkyl where the heteroaryl moiety contains 1 to 4 heteroatoms selected from N, O and S as ring-constituting atoms, and the alkylene moiety has 1 to 5 carbon atoms; cycloalkylalkyl where the cycloalkyl moiety has 3 to 6 carbon atoms, and the alkylene moiety has 1 to 5 carbon atoms; or $C_{2-6}$ alkenyl,

X represents O or $CH_2$, and

Y represents C(=O),

provided that the $C_{1-10}$ alkyl as $R^1$; the alkylene moiety and cycloalkyl moiety of the cycloalkylalkyl where the cycloalkyl moiety has 3 to 6 carbon atoms, and the alkylene moiety has 1 to 5 carbon atoms as $R^1$; the alkylene moiety of the aralkyl where the aryl moiety has 6 to 10 carbon atoms, and the alkylene moiety has 1 to 5 carbon atoms as $R^1$; and the alkylene moiety of the heteroarylalkyl where the heteroaryl moiety contains 1 to 4 heteroatoms selected from N, O and S as ring-constituting atoms, and the alkylene moiety has 1 to 5 carbon atoms as $R^1$ may be substituted with at least one substituent selected from

1 to 6 halogens; hydroxy; $C_{1-6}$ alkoxy; $C_{6-10}$ aryloxy; $C_{1-6}$ alkanoyl; $C_{1-6}$ alkanoyloxy; carboxyl; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; alkylsulfonyl where the alkyl moiety has 1 to 6 carbon atoms; aminosulfonyl; alkylsulfinyl where the alkyl moiety has 1 to 6 carbon atoms; alkylthio where the alkyl moiety has 1 to 6 carbon atoms; $C_{1-6}$ alkoxy substituted with 1 to 6 halogens; and arylcarbonyl where the aryl moiety has 6 to 10 carbon atoms,

the $C_{6-10}$ aryl as $R^1$; the aryl moiety of the aralkyl where the aryl moiety has 6 to 10 carbon atoms, and the alkylene moiety has 1 to 5 carbon atoms as $R^1$; the aryl moiety of the $C_{6-10}$ aryloxy as $R^3$, $R^4$, or $R^5$; the aryl moiety of the $C_{6-10}$ arylamino as $R^3$, $R^4$, or $R^5$; the $C_{6-10}$ aryl as $R^9$ or $R^{10}$; the heteroaryl containing 1 to 4 heteroatoms selected from N, O and S as ring-constituting atoms as $R^9$ or $R^{10}$; the aryl moiety of the aralkyl where the aryl moiety has 6 to 10 carbon atoms, and the alkylene moiety has 1 to 5 carbon atoms as $R^9$ or $R^{10}$; and the heteroaryl moiety of the heteroarylalkyl where the heteroaryl moiety contains 1 to 4 heteroatoms selected from N, O and S as ring-constituting atoms, and the alkylene moiety has 1 to 5 carbon atoms as $R^9$ or $R^{10}$ may be substituted with at least one substituent selected from

$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy,

the heterocyclic ring as $R^2$ may have, besides the oxo group, the substituents that the $C_{6-10}$ aryl as $R^1$ mentioned above may have, and pyridine 1-oxide as $R^2$ may have the substituents that the $C_{6-10}$ aryl as $R^1$ mentioned above may have,

when $R^1$ is $C_{1-10}$ alkyl, it may be substituted with $NR^{11}R^{12}$, where $R^{11}$ and $R^{12}$, which are the same or different, represent hydrogen; $C_{1-10}$ alkyl; or aralkyl where the aryl moiety has 6 to 10 carbon atoms, and the alkylene moiety has 1 to 5 carbon atoms; or $R^{11}$, $R^{12}$, the nitrogen atom to which $R^{11}$ and $R^{12}$ bind, and optionally, 1 or 2 heteroatoms may combine together to form a 5- to 7-membered ring, and the alkylene moiety of the aralkyl where the aryl moiety has 6 to 10 carbon atoms, and the alkylene moiety has 1 to 5 carbon atoms as $R^1$ may be substituted with at least one substituent selected from phenyl, and $C_{1-6}$ alkyl substituted with 1 to 3 halogens),

or pharmaceutically acceptable salt is the pharmaceutical composition for treatment or prevention according to any one of [1] to [12];

[14] The pharmaceutical composition for treatment or prevention according to [13], wherein the compound represented by the general formula (I) is a compound in which $R^1$ represents $C_{1-10}$ alkyl; cycloalkylalkyl where the

cycloalkyl moiety has 3 to 6 carbon atoms, and the alkylene moiety has 1 to 5 carbon atoms; or aralkyl where the aryl moiety has 6 to 10 carbon atoms, and the alkylene moiety has 1 to 5 carbon atoms;

[15] The pharmaceutical composition for treatment or prevention according to [13] or [14], wherein the compound represented by the general formula (I) is a compound in which $R^1$ represents cycloalkylalkyl where the cycloalkyl moiety has 3 to 6 carbon atoms, and the alkylene moiety has 1 to 5 carbon atoms;

[16] The pharmaceutical composition for treatment or prevention according to [13], wherein the compound represented by the general formula (I) is a compound in which $R^1$ represents $C_{2-6}$ alkyl substituted with hydroxy; $C_{1-6}$ alkyl substituted with 1 to 6 halogens; or $C_{2-6}$ alkyl substituted with $C_{1-6}$ alkoxy;

[17] The pharmaceutical composition for treatment or prevention according to [13], wherein the compound represented by the general formula (I) is a compound in which $R^1$ represents allyl, fluoropropyl, 2-(pyridin-3-yl)ethyl, 2-(methylsulfonyl)ethyl, or 2-(aminosulfonyl)ethyl;

[18] The pharmaceutical composition for treatment or prevention according to any one of [13] to [17], wherein the compound represented by the general formula (I) is a compound in which $R^2$ represents a 5- to 7-membered heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or the heterocyclic ring condensed with a benzene ring; or pyridine 1-oxide;

[19] The pharmaceutical composition for treatment or prevention according to any one of [13] to [18], wherein the compound represented by the general formula (1) is a compound in which $R^2$ represents pyridine 1-oxide which may be substituted with 1 to 4 substituents selected from $C_{1-10}$ alkyl substituted with 1 to 3 fluorine atoms and unsubstituted $C_{1-10}$ alkyl;

[20] The pharmaceutical composition for treatment or prevention according to any one of [13] to [19], wherein the compound represented by the general formula (1) is a compound in which $R^2$ represents pyridine 1-oxide;

[21] The pharmaceutical composition for treatment or prevention according to any one of [13] to [18], wherein the compound represented by the general formula (I) is a compound in which $R^2$ represents pyridin-2(1H)-one which may be substituted with 1 to 4 substituents selected from $C_{1-10}$ alkyl substituted with 1 to 3 fluorine atoms and unsubstituted $C_{1-10}$ alkyl;

[22] The pharmaceutical composition for treatment or prevention according to any one of [13] to [18] and [21], wherein the compound represented by the general formula (I) is a compound in which $R^2$ represents pyridin-2(1H)-one; 1-$C_{1-6}$ alkylpyridin-2(1H)-one; or 6-$C_{1-6}$ alkylpyridin-2(1H)-one;

[23] The pharmaceutical composition for treatment or prevention according to any one of [13] to [18], wherein the compound represented by the general formula (I) is a compound in which $R^2$ represents pyridin-4(1H)-one which may be substituted with 1 to 4 substituents selected from $C_{1-10}$ alkyl substituted with 1 to 3 fluorine atoms and unsubstituted $C_{1-10}$ alkyl;

[24] The pharmaceutical composition for treatment or prevention according to any one of [13] to [18] and [23], wherein the compound represented by the general formula (1) is a compound in which $R^2$ represents pyridin-4(1H)-one or 1-$C_{1-6}$ alkylpyridin-4(1H)-one;

[25] The pharmaceutical composition for treatment or prevention according to any one of [13] to [18], wherein the compound represented by the general formula (I) is a compound in which $R^2$ represents pyridazin-3(2H)-one which may be substituted with 1 to 3 substituents selected from $C_{1-10}$ alkyl substituted with 1 to 3 fluorine atoms and unsubstituted $C_{1-10}$ alkyl;

[26] The pharmaceutical composition for treatment or prevention according to any one of [13] to [18] and [25], wherein the compound represented by the general formula (I) is a compound in which $R^2$ represents pyridazin-3(2H)-one;

[27] The pharmaceutical composition for treatment or prevention according to any one of [13] to [18], wherein the compound represented by the general formula (I) is a compound in which $R^2$ represents pyrazin-2(1H)-one which may be substituted with 1 to 3 substituents selected from $C_{1-10}$ alkyl, and $C_{1-10}$ alkyl substituted with 1 to 3 fluorine atoms;

[28] The pharmaceutical composition for treatment or prevention according to any one of [13] to [18] and [27], wherein the compound represented by the general formula (1) is a compound in which $R^2$ represents pyrazin-2(1H)-one;

[29] The pharmaceutical composition for treatment or prevention according to any one of [13] to [18], wherein the compound represented by the general formula (1) is a compound in which $R^2$ represents 4H-pyran-4-one or 2H-pyran-2-one which may be substituted with 1 to 3 substituents selected from $C_{1-10}$ alkyl substituted with 1 to 3 fluorine atoms and unsubstituted $C_{1-10}$ alkyl;

[30] The pharmaceutical composition for treatment or prevention according to any one of [13] to [18] and [29], wherein the compound represented by the general formula (I) is a compound in which $R^2$ represents 4H-pyran-4-one or 2H-pyran-2-one;

[31] The pharmaceutical composition for treatment or prevention according to any one of [13] to [18], wherein the compound represented by the general formula (I) is a compound in which $R^2$ represents quinolin-2(1H)-one which

may be substituted with 1 to 3 substituents selected from $C_{1-10}$ alkyl substituted with 1 to 3 fluorine atoms and unsubstituted $C_{1-10}$ alkyl;

[32] The pharmaceutical composition for treatment or prevention according to any one of [13] to [18] and [31], wherein the compound represented by the general formula (I) is a compound in which $R^2$ represents quinolin-2(1H)-one;

[33] The pharmaceutical composition for treatment or prevention according to any one of [13] to [18], wherein the compound represented by the general formula (I) is a compound in which $R^2$ represents pyrimidin-4(3H)-one or pyrimidin-2,4(1H,3H)-dione which may be substituted with 1 to 3 substituents selected from $C_{1-10}$ alkyl substituted with 1 to 3 fluorine atoms and unsubstituted $C_{1-10}$ alkyl;

[34] The pharmaceutical composition for treatment or prevention according to any one of [13] to [18] and [33], wherein the compound represented by the general formula (I) is a compound in which $R^2$ represents pyrimidin-4(3H)-one or pyrimidin-2,4(1H,3H)-dione;

[35] The pharmaceutical composition for treatment or prevention according to any one of [13] to [34], wherein the compound represented by the general formula (I) is a compound in which X represents $CH_2$;

[36] The pharmaceutical composition for treatment or prevention according to any one of [13] to [35], wherein the compound represented by the general formula (1) is a compound in which one of $R^3$ and $R^4$ represents hydroxy and the other represents hydrogen;

[37] The pharmaceutical composition for treatment or prevention according to any one of [13] to [35], wherein the compound represented by the general formula (I) is a compound in which $R^3$ represents halogen; cyano; carbamoyl; $C_{1-6}$ alkoxy; $C_{1-6}$ alkanoyloxy; amino; or acylamino where the acyl moiety has 2 to 6 carbon atoms; $R^4$ represents hydrogen or hydroxy; and $R^5$ represents hydrogen;

[38] The pharmaceutical composition for treatment or prevention according to any one of [13] to [35], wherein the compound represented by the general formula (I) is a compound in which $R^3$ represents hydroxy; carbamoyl; or $C_{1-6}$ alkanoyloxy; $R^4$ represents hydrogen; and $R^5$ represents hydrogen;

[39] The pharmaceutical composition for treatment or prevention according to any one of [13] to [35], wherein the compound represented by the general formula (1) is a compound in which $R^3$ represents hydroxy; $R^4$ represents hydrogen; and $R^5$ represents hydrogen;

[40] The pharmaceutical composition for treatment or prevention according to any one of [13] to [35], wherein the compound represented by the general formula (I) is a compound in which $R^3$, $R^4$ and $R^5$ all represent hydrogen;

[41] The pharmaceutical composition for treatment or prevention according to any one of [13] to [40], wherein the compound represented by the general formula (1) is a compound in which $R^{6a}$, $R^{6b}$, $R^7$, $R^8$, $R^9$ and $R^{10}$ all represent hydrogen;

[42] The pharmaceutical composition for treatment or prevention according to [13], wherein the compound represented by the general formula (I) is a compound in which

$R^5$, $R^{6a}$, $R^{6b}$, $R^7$, $R^8$, $R^9$ and $R^{10}$ all represent hydrogen,

$R^1$ represents hydrogen; $C_{1-6}$ alkyl; $C_{2-6}$ alkenyl; cycloalkylalkyl where the cycloalkyl moiety has 3 to 6 carbon atoms, and the alkylene moiety has 1 to 5 carbon atoms; or aralkyl where the aryl moiety has 6 to 10 carbon atoms, and the alkylene moiety has 1 to 5 carbon atoms,

$R^2$ represents a 5- to 7-membered heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or the heterocyclic ring condensed with a benzene ring; or pyridine 1-oxide,

$R^2$ binds to Y via a carbon atom as a ring-constituting atom of $R^2$,

$R^3$ and $R^4$, which are the same or different, represent hydrogen; hydroxy; halogen; cyano; carbamoyl; $C_{1-6}$ alkoxy; $C_{6-10}$ aryloxy; $C_{1-6}$ alkanoyloxy; amino; or acylamino where the acyl moiety has 2 to 6 carbon atoms,

X represents $CH_2$, and

Y represents C(=O),

provided that the $C_{1-6}$ alkyl as $R^1$; the alkylene moiety and cycloalkyl moiety of the cycloalkylalkyl where the cycloalkyl moiety has 3 to 6 carbon atoms, and the alkylene moiety has 1 to 5 carbon atoms as $R^1$; or the alkylene moiety of the aralkyl where the aryl moiety has 6 to 10 carbon atoms, and the alkylene moiety has 1 to 5 carbon atoms as $R^1$ may be substituted with at least one substituent selected from:

1 to 6 halogens; hydroxy; $C_{1-6}$ alkoxy; $C_{6-10}$ aryloxy; $C_{1-6}$ alkanoyl; $C_{1-6}$ alkanoyloxy; carboxyl; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; alkylsulfonyl where the alkyl moiety has 1 to 6 carbon atoms; aminosulfonyl; alkylsulfinyl where the alkyl moiety has 1 to 6 carbon atoms; alkylthio where the alkyl moiety has 1 to 6 carbon atoms; $C_{1-6}$ alkoxy substituted with 1 to 6 halogens; and arylcarbonyl where the aryl moiety has 6 to 10 carbon atoms,

the aryl moiety of the aralkyl where the aryl moiety has 6 to 10 carbon atoms, and the alkylene moiety has 1 to

5 carbon atoms as $R^1$; or the aryl moiety of the $C_{6-10}$ aryloxy as $R^3$ and $R^4$ may be substituted with at least one substituent selected from: $C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy,

the heterocyclic ring as $R^2$ may have, besides the oxo group, the substituents that the aryl moiety of the aralkyl where the aryl moiety has 6 to 10 carbon atoms, and the alkylene moiety has 1 to 5 carbon atoms as $R^1$ mentioned above may have, and pyridine 1-oxide as $R^2$ may have the substituents that the aryl moiety of the aralkyl where the aryl moiety has 6 to 10 carbon atoms, and the alkylene moiety has 1 to 5 carbon atoms as $R^1$ mentioned above may have, and

the alkylene moiety of the aralkyl where the aryl moiety has 6 to 10 carbon atoms, and the alkylene moiety has 1 to 5 carbon atoms as $R^1$ may be substituted with at least one substituent selected from phenyl, and $C_{1-6}$ alkyl substituted with 1 to 3 halogens;

[43] The pharmaceutical composition for treatment or prevention according to [13] or [42], wherein the compound represented by the general formula (I) is a compound in which $R^1$ represents $C_{1-6}$ alkyl; cycloalkylalkyl where the cycloalkyl moiety has 3 to 6 carbon atoms, and the alkylene moiety has 1 to 5 carbon atoms; or aralkyl where the aryl moiety has 6 to 10 carbon atoms, and the alkylene moiety has 1 to 5 carbon atoms;

[44] The pharmaceutical composition for treatment or prevention according to [13], [42] or [43], wherein the compound represented by the general formula (I) is a compound in which $R^1$ represents a cycloalkylalkyl where the cycloalkyl moiety has 3 to 6 carbon atoms, and the alkylene moiety has 1 to 5 carbon atoms;

[45] The pharmaceutical composition for treatment or prevention according to [13] or [42], wherein the compound represented by the general formula (I) is a compound in which $R^1$ represents $C_{2-6}$ alkyl substituted with hydroxy; $C_{1-6}$ alkyl substituted with 1 to 6 halogens; or $C_{2-6}$ alkyl substituted with $C_{1-6}$ alkoxy;

[46] The pharmaceutical composition for treatment or prevention according to [13] or [42], wherein the compound represented by the general formula (I) is a compound in which $R^1$ represents allyl, fluoropropyl, 2-(pyridin-3-yl)ethyl, 2-(methylsulfonyl)ethyl, or 2-(aminosulfonyl)ethyl;

[47] The pharmaceutical composition for treatment or prevention according to any one of [13] and [42] to [46], wherein the compound represented by the general formula (1) is a compound in which $R^2$ represents pyridine 1-oxide, pyridin-2(1H)-one, pyridin-4(1H)-one, pyridazin-3(2H)-one, pyrazin-2(1H)-one, 4H-pyran-4-one, 2H-pyran-2-one, quinolin-2(1H)-one, pyrimidin-4(3H)-one or pyrimidin-2,4(1H,3H)-dione, which may be substituted with a substituent selected from $C_{1-10}$ alkyl substituted with 1 to 3 fluorine atoms and unsubstituted $C_{1-10}$ alkyl;

[48] The pharmaceutical composition for treatment or prevention according to any one of [13] and [42] to [47], wherein the compound represented by the general formula (I) is a compound in which $R^2$ represents pyridine 1-oxide which may be substituted with 1 to 4 substituents selected from $C_{1-10}$ alkyl and $C_{1-10}$ alkyl substituted with 1 to 3 fluorine atoms;

[49] The pharmaceutical composition for treatment or prevention according to any one of [13] and [42] to [48], wherein the compound represented by the general formula (1) is a compound in which $R^2$ represents pyridine 1-oxide;

[50] The pharmaceutical composition for treatment or prevention according to any one of [13] and [42] to [47], wherein the compound represented by the general formula (I) is a compound in which $R^2$ represents pyridin-2(1H)-one which may be substituted with 1 to 4 substituents selected from $C_{1-10}$ alkyl and $C_{1-10}$ alkyl substituted with 1 to 3 fluorine atoms;

[51] The pharmaceutical composition for treatment or prevention according to any one of [13] and [42] to [47], wherein the compound represented by the general formula (1) is a compound in which $R^2$ represents pyridin-2(1H)-one; 1-$C_{1-6}$ alkylpyridin-2(1H)-one; or 6-$C_{1-6}$ alkylpyridin-2(1H)-one;

[52] The pharmaceutical composition for treatment or prevention according to any one of [13] and [42] to [47], wherein the compound represented by the general formula (I) is a compound in which $R^2$ represents pyridin-4(1H)-one which may be substituted with 1 to 4 substituents selected from $C_{1-10}$ alkyl substituted with 1 to 3 fluorine atoms and unsubstituted $C_{1-10}$ alkyl;

[53] The pharmaceutical composition for treatment or prevention according to any one of [13], [42] to [47] and [52], wherein the compound represented by the general formula (I) is a compound in which $R^2$ represents pyridin-4(1H)-one or 1-$C_{1-6}$ alkylpyridin-4(1H)-one;

[54] The pharmaceutical composition for treatment or prevention according to any one of [13] and [42] to [47], wherein the compound represented by the general formula (I) is a compound in which $R^2$ represents pyridazin-3(2H)-one which may be substituted with 1 to 3 substituents selected from $C_{1-10}$ alkyl substituted with 1 to 3 fluorine atoms and unsubstituted $C_{1-10}$ alkyl;

[55] The pharmaceutical composition for treatment or prevention according to any one of [13], [42] to [47] and [54], wherein the compound represented by the general formula (1) is a compound in which $R^2$ represents pyridazin-3(2H)-one;

[56] The pharmaceutical composition for treatment or prevention according to any one of [13] and [42] to [47], wherein the compound represented by the general formula (I) is a compound in which $R^2$ represents pyrazin-2(1H)-one which may be substituted with 1 to 3 substituents selected from $C_{1-10}$ alkyl substituted with 1 to 3 fluorine atoms and unsubstituted $C_{1-10}$ alkyl;

[57] The pharmaceutical composition for treatment or prevention according to any one of [13], [42] to [47] and [56], wherein the compound represented by the general formula (I) is a compound in which $R^2$ represents pyrazin-2(1H)-one;

[58] The pharmaceutical composition for treatment or prevention according to any one of [13] and [42] to [47], wherein the compound represented by the general formula (1) is a compound in which $R^2$ represents 4H-pyran-4-one or 2H-pyran-2-one which may be substituted with 1 to 3 substituents selected from $C_{1-10}$ alkyl substituted with 1 to 3 fluorine atoms and unsubstituted $C_{1-10}$ alkyl;

[59] The pharmaceutical composition for treatment or prevention according to any one of [13], [42] to [47] and [58], wherein the compound represented by the general formula (1) is a compound in which $R^2$ represents 4H-pyran-4-one or 2H-pyran-2-one;

[60] The pharmaceutical composition for treatment or prevention according to any one of [13] and [42] to [47], wherein the compound represented by the general formula (1) is a compound in which $R^2$ represents quinolin-2(1H)-one which may be substituted with 1 to 3 substituents selected from $C_{1-10}$ alkyl substituted with 1 to 3 fluorine atoms and unsubstituted $C_{1-10}$ alkyl;

[61] The pharmaceutical composition for treatment or prevention according to any one of [13], [42] to [47] and [60], wherein the compound represented by the general formula (1) is a compound in which $R^2$ represents quinolin-2(1H)-one;

[62] The pharmaceutical composition for treatment or prevention according to any one of [13] and [42] to [47], wherein the compound represented by the general formula (I) is a compound in which $R^2$ represents pyrimidin-4(3H)-one or pyrimidin-2,4(1H,3H)-dione, which may be substituted with 1 to 3 substituents selected from $C_{1-10}$ alkyl substituted with 1 to 3 fluorine atoms and unsubstituted $C_{1-10}$ alkyl;

[63] The pharmaceutical composition for treatment or prevention according to any one of [13], [42] to [47] and [62], wherein the compound represented by the general formula (I) is a compound in which $R^2$ represents pyrimidin-4(3H)-one or pyrimidin-2,4(1H,3H)-dione;

[64] The pharmaceutical composition for treatment or prevention according to any one of [13], [42] to [63], wherein the compound represented by the general formula (1) is a compound in which one of $R^3$ and $R^4$ represents hydroxy and the other represents hydrogen;

[65] The pharmaceutical composition for treatment or prevention according to any one of [13], [42] to [63], wherein the compound represented by the general formula (I) is a compound in which $R^3$ represents halogen; cyano; carbamoyl; $C_{1-6}$ alkoxy; $C_{1-6}$ alkanoyloxy; amino; or acylamino where the acyl moiety has 2 to 6 carbon atoms; and $R^4$ represents hydrogen or hydroxy;

[66] The pharmaceutical composition for treatment or prevention according to any one of [13], [42] to [63], wherein the compound represented by the general formula (I) is a compound in which $R^3$ represents hydroxy; carbamoyl; or $C_{1-6}$ alkanoyloxy; and $R^4$ represents hydrogen;

[67] The pharmaceutical composition for treatment or prevention according to any one of [13], [42] to [63], wherein the compound represented by the general formula (1) is a compound in which $R^3$ represents hydroxy and $R^4$ represents hydrogen;

[68] The pharmaceutical composition for treatment or prevention according to any one of [13], [42] to [63], wherein the compound represented by the general formula (I) is a compound in which $R^3$ and $R^4$ both represent hydrogen;

[69] The pharmaceutical composition for treatment or prevention according to [13], wherein the compound represented by the general formula (I) is a compound represented by the following general formula (II):

[Formula 2]

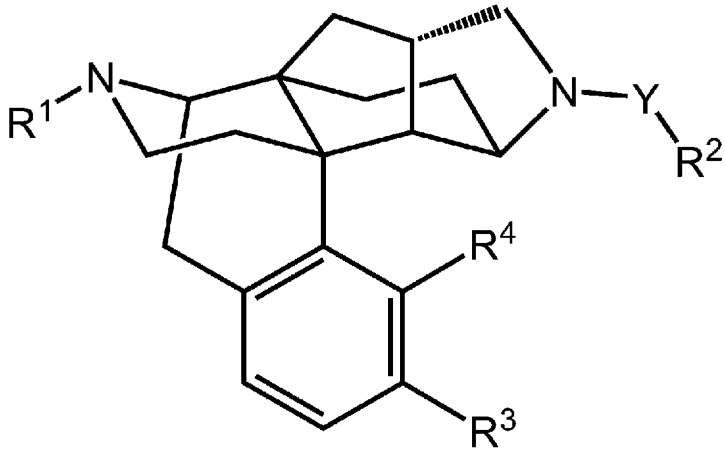

(I I)

(wherein $R^1$ represents hydrogen; $C_{1-6}$ alkyl; or cycloalkylalkyl where the cycloalkyl moiety has 3 to 6 carbon atoms, and the alkylene moiety has 1 to 5 carbon atoms;

$R^2$ represents a 5- to 7-membered heterocyclic ring containing 1 or 2 nitrogen atoms and at least one carbon atom as ring-constituting atoms, containing at least one set of adjacent ring-constituting atoms bound by a double bond, and further substituted with at least one oxo group; or pyridine 1-oxide;
$R^2$ binds to Y via a carbon atom as a ring-constituting atom of $R^2$,
$R^3$ and $R^4$, which are the same or different, represent hydrogen; hydroxy; or $C_{1-6}$ alkoxy,
Y represents C(=O),
provided that the $C_{1-6}$ alkyl as $R^1$; and the alkylene moiety and cycloalkyl moiety of the cycloalkylalkyl where the cycloalkyl moiety has 3 to 6 carbon atoms and the alkylene moiety has 1 to 5 carbon atoms as $R^1$ may be substituted with at least one substituent selected from:

1 to 6 halogens; hydroxy; and $C_{1-6}$ alkoxy,
the heterocyclic ring as $R^2$ may have, besides the oxo group, at least one substituent selected from:

$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy, and
the pyridine 1-oxide as $R^2$ may have at least one substituent selected from:
$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy);

[70] The pharmaceutical composition for treatment or prevention according to [69], wherein the compound represented by the general formula (II) is a compound in which $R^1$ is cycloalkylalkyl where the cycloalkyl moiety has 3 to 6 carbon atoms, and the alkylene moiety has 1 to 5 carbon atoms;
[71] The pharmaceutical composition for treatment or prevention according to [69] or [70], wherein the compound represented by the general formula (II) is a compound in which $R^2$ is represented by a formula (III):

[Formula 3]

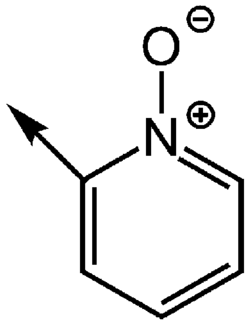

(III)

a formula (IV):

[Formula 4]

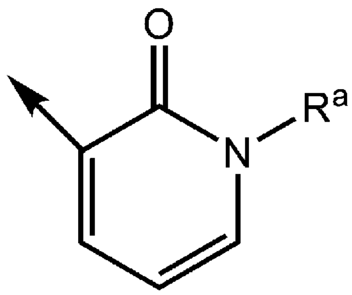

(IV)

a formula (V):

[Formula 5]

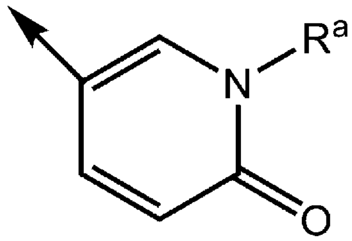

(V)

or a formula (VI):

[Formula 6]

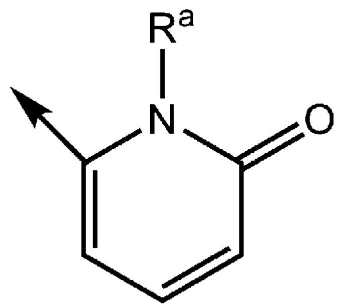

(VI)

(wherein in formulas (III) to (VI), $R^a$ represents hydrogen or $C_{1-6}$ alkyl, and binding to Y occurs at the position of the arrow);

[72] The pharmaceutical composition for treatment or prevention according to any one of [69] to [71], wherein the compound represented by the general formula (II) is a compound in which $R^3$ is hydroxy;

[73] The pharmaceutical composition for treatment or prevention according to any one of [69] to [72], wherein the compound represented by the general formula (II) is a compound in which $R^4$ is hydrogen;

[74] The pharmaceutical composition for treatment or prevention according to [13], wherein the compound represented by the general formula (I) is at least one compound selected from:

2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
5-((1S,3aR,5aS,6R,11bR,11cS)-14-(cyclopropylmethyl)-10-hydroxy-2,3,3a,4,5,6,7,11c-octahydro-1H-6,1b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-6-methylpyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrinudine-2,4(1H,3H)-dione,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-4(1H)-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-4(1H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1 -methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridazin-3(2H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)quinolin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-2H-pyran-2-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-4H-pyran-4-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-4(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrazin-2(1H)-one,
2-((1S,3aR,5aS,6R,11bR,11cS)-10-acetoxy-14-(cyclopropylmethyl)-2,3,3a,4,5,6,7,11c-octahydro-1H-6,11b-(epiminoethano)-1,5a-methanonaphtho[1,2-e]indole-3-carbonyl)pyridine 1-oxide,
6-((1S,3aR,5aS,6R,11bR,11cS)-14-(cyclopropylmethyl)-2,3,3a,4,5,6,7,11c-octahydro-1H-6, I lb-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrazin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epimnoethano)-1,5a-methanonaphtho[1,2-e]indole-3-carbonyl)pyrimidine-2,4(1H,3H)-dione,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidin-4(3H)-one, and
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one;

[75] The pharmaceutical composition for treatment or prevention according to [13], wherein the compound represented by the general formula (I) is at least one compound selected from:

6-((1S,3aR,5aS,6R,11bR,11cS)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methyl-1,2-dihydro-3H-pyrazol-3-one,
5-chloro-3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1,3-dimethylpyrimidine-2,4(1H,3H)-dione, and
6-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e]indole-3-carbonyl)pyridin-2(1H)-one;

[76] The pharmaceutical composition for treatment or prevention according to any one of [13] and [69] to [73], wherein the compound represented by the general formula (1) is at least one compound selected from:

2-((1S,3aR,5aS,6R,11bR, 11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one, and
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one.

Further, the present invention has the following aspects.

<1> A pharmaceutical composition for the treatment or prevention of stress-related disorders, stress-induced anxiety disorders, or stress-related disorders or anxiety disorders associated with depression, the composition containing

a selective δ-opioid receptor agonist as an active component;

<2> The pharmaceutical composition for treatment or prevention according to <1>, wherein the selective δ-opioid receptor agonist also has a κ-opioid receptor antagonist action;

<3> The pharmaceutical composition for treatment or prevention according to <1> or <2>, wherein the selective δ-opioid receptor agonist also has a μ-opioid receptor antagonist action and a κ-opioid receptor antagonist action;

<4> The pharmaceutical composition for treatment or prevention according to any one of <1> to <3>, wherein the stress-related disorder is an acute stress disorder, post-traumatic stress disorder, or adjustment disorder;

<5> The pharmaceutical composition for treatment or prevention according to any one of <1> to <4>, wherein the stress-related disorder is an acute stress disorder or post-traumatic stress disorder;

<6> The pharmaceutical composition for treatment or prevention according to <4> or <5> which functions as a pharmaceutical composition for the treatment or prevention of post-traumatic stress disorder, and has an inhibitory effect on intrusion symptoms related to the traumatic event, an inhibitory effect on persistent avoidance of stimuli associated with the traumatic event, or an inhibitory effect on negative changes in cognitions and mood related to the traumatic event, that begin following the traumatic event;

<7> The pharmaceutical composition for treatment or prevention according to <6>, wherein the intrusion symptoms related to the traumatic event that begin following the traumatic event include at least one symptom among (1) to (5) described below:

(1) recurrent, involuntary and intrusive painful recollections of the traumatic event;
(2) recurrent distressing dreams in which dream content and/or emotions are related to the traumatic event;
(3) dissociative symptoms causing feelings or actions of the traumatic event reoccurring;
(4) intense or prolonged psychological pain upon exposure to internal or external cues that symbolize aspects of the traumatic event or something similar; and
(5) clear physiological reactions to internal or external cues that symbolize aspects of the traumatic event or something similar;

<8> The pharmaceutical composition for treatment or prevention according to any one of <1> to <7>, wherein the composition suppresses flashbacks;

<9> The pharmaceutical composition for treatment or prevention according to any one of <1> to <8>, wherein the composition suppresses avoidance of events or matters related to a trauma;

<10> The pharmaceutical composition for treatment or prevention according to any one of <1> to <9>, wherein the composition has a facilitation effect on the extinction of memories of a traumatic event, and/or an inhibitory effect on the reconsolidation of memories of a traumatic event;

<11> The pharmaceutical composition for treatment or prevention according to <10>, wherein the facilitation effect on the extinction of memories of a traumatic event is a facilitation effect on extinction learning of memories of a traumatic event;

<12> The pharmaceutical composition for treatment or prevention according to any one of <1> to <11>, wherein the composition palliates fear from fear memories;

<13> The pharmaceutical composition for treatment or prevention according to any one of <1> to <12>, wherein the selective δ-opioid receptor agonist is a compound represented by the following general formula (I):

[Formula 7]

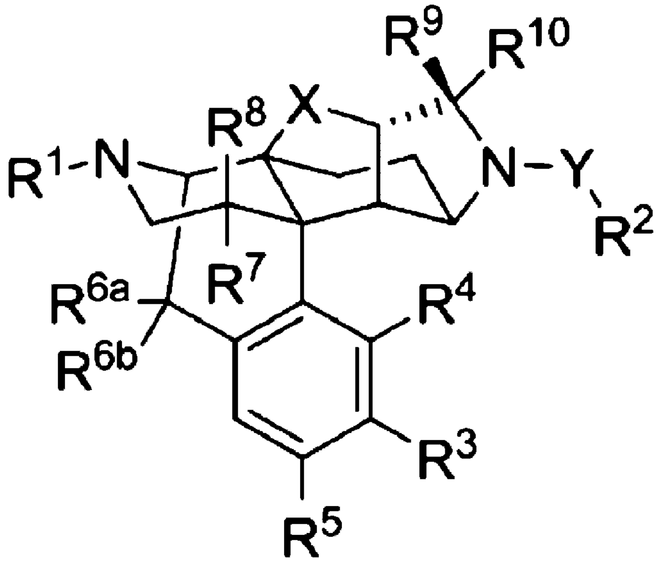

( I )

(wherein $R^1$ represents hydrogen; $C_{1-10}$ alkyl; $C_{6-10}$ aryl; $C_{2-6}$ alkenyl; cycloalkylalkyl where the cycloalkyl moiety has 3 to 6 carbon atoms, and the alkylene moiety has 1 to 5 carbon atoms; aralkyl where the aryl moiety has 6 to 10 carbon atoms, and the alkylene moiety has 1 to 5 carbon atoms; $C_{3-6}$ cycloalkyl; or heteroarylalkyl where the heteroaryl moiety contains 1 to 4 heteroatoms selected from N, O and S as ring-constituting atoms, and the alkylene moiety has 1 to 5 carbon atoms,

$R^2$ represents heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or pyridine 1-oxide,
$R^2$ binds to Y via a carbon atom as a ring-constituting atom of $R^2$,
R3, $R^4$, and $R^5$, which are the same or different, represent hydrogen; hydroxy; halogen; cyano; carbamoyl; $C_{1-6}$ alkoxy; $C_{6-10}$ aryloxy; $C_{1-6}$ alkanoyloxy; nitro; amino; $C_{1-8}$ alkylamino; $C_{6-10}$ arylamino; or acylamino where the acyl moiety has 2 to 6 carbon atoms,
$R^{6a}$ and $R^{6b}$, which are the same or different, represent hydrogen; fluorine; or hydroxy, or $R^{6a}$ and $R^{6b}$ combine together to represent =O,
$R^7$ and $R^8$, which are the same or different, represent hydrogen; fluorine; or hydroxy,
$R^9$ and $R^{10}$, which are the same or different, represent hydrogen; $C_{1-6}$ alkyl; $C_{6-10}$ aryl; heteroaryl containing 1 to 4 heteroatoms selected from N, O and S as ring-constituting atoms; aralkyl where the aryl moiety has 6 to 10 carbon atoms, and the alkylene moiety has 1 to 5 carbon atoms; heteroarylalkyl where the heteroaryl moiety contains 1 to 4 heteroatoms selected from N, O and S as ring-constituting atoms, and the alkylene moiety has 1 to 5 carbon atoms; cycloalkylalkyl where the cycloalkyl moiety has 3 to 6 carbon atoms, and the alkylene moiety has 1 to 5 carbon atoms; or $C_{2-6}$ alkenyl,
X represents O or $CH_2$, and
Y represents C(=O),
provided that the $C_{1-10}$ alkyl as $R^1$; the alkylene moiety and cycloalkyl moiety of the cycloalkylalkyl where the cycloalkyl moiety has 3 to 6 carbon atoms, and the alkylene moiety has 1 to 5 carbon atoms as $R^1$; the alkylene moiety of the aralkyl where the aryl moiety has 6 to 10 carbon atoms, and the alkylene moiety has 1 to 5 carbon atoms as $R^1$; and the alkylene moiety of the heteroarylalkyl where the heteroaryl moiety contains 1 to 4 heteroatoms selected from N, O and S as ring-constituting atoms, and the alkylene moiety has 1 to 5 carbon atoms as $R^1$ may be substituted with at least one substituent selected from:

1 to 6 halogens; hydroxy; $C_{1-6}$ alkoxy; $C_{6-10}$ aryloxy; $C_{1-6}$ alkanoyl; $C_{1-6}$ alkanoyloxy; carboxyl; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; alkylsulfonyl where the alkyl moiety has 1 to 6 carbon atoms; aminosulfonyl; alkylsulfinyl where the alkyl moiety has 1 to 6 carbon atoms; alkylthio where the alkyl moiety has 1 to 6 carbon atoms; $C_{1-6}$ alkoxy substituted with

1 to 6 halogens; and arylcarbonyl where the aryl moiety has 6 to 10 carbon atoms,
the $C_{6-10}$ aryl as $R^1$; the aryl moiety of the aralkyl where the aryl moiety has 6 to 10 carbon atoms, and the alkylene moiety has 1 to 5 carbon atoms as $R^1$; the aryl moiety of the $C_{6-10}$ aryloxy as $R^3$, $R^4$, or $R^5$; the aryl moiety of the $C_{6-10}$ arylamino as $R^3$, $R^4$, or $R^5$; the $C_{6-10}$ aryl as $R^9$ or $R^{10}$; the heteroaryl containing 1 to 4 heteroatoms selected from N, O and S as ring-constituting atoms as $R^9$ or $R^{10}$; the aryl moiety of the aralkyl where the aryl moiety has 6 to 10 carbon atoms, and the alkylene moiety has 1 to 5 carbon atoms as $R^9$ or $R^{10}$; and the heteroaryl moiety of the heteroarylalkyl where the heteroaryl moiety contains 1 to 4 heteroatoms selected from N, O and S as ring-constituting atoms, and the alkylene moiety has 1 to 5 carbon atoms as $R^9$ or $R^{10}$ may be substituted with at least one substituent selected from:

$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy,
the heterocyclic ring as $R^2$ may have, besides the oxo group, the substituents that the $C_{6-10}$ aryl as $R^1$ mentioned above may have, and pyridine 1-oxide as $R^2$ may have the substituents that the $C_{6-10}$ aryl as $R^1$ mentioned above may have,
when $R^1$ is $C_{1-10}$ alkyl, it may be substituted with $NR^{11}R^{12}$, where $R^{11}$ and $R^{12}$, which are the same or different, represent hydrogen; $C_{1-10}$ alkyl; or aralkyl where the aryl moiety has 6 to 10 carbon atoms, and the alkylene moiety has 1 to 5 carbon atoms; or $R^{11}$, $R^{12}$, the nitrogen atom to which $R^{11}$ and $R^{12}$ bind, and optionally, 1 or 2 heteroatoms may combine together to form a 5- to 7-membered ring, and the alkylene moiety of the aralkyl where the aryl moiety has 6 to 10 carbon atoms, and the alkylene moiety has 1 to 5 carbon atoms as $R^1$ may be substituted with at least one substituent selected from phenyl, and $C_{1-6}$ alkyl substituted with 1 to 3 halogens),
or a pharmaceutically acceptable salt thereof;

<14> The pharmaceutical composition for treatment or prevention according to <13>, wherein the compound represented by the general formula (I) is a compound in which

$R^5$, $R^{6a}$, $R^{6b}$, $R^7$, $R^8$, $R^9$ and $R^{10}$ all represent hydrogen,
$R^1$ represents hydrogen; $C_{1-6}$ alkyl; $C_{2-6}$ alkenyl; cycloalkylalkyl where the cycloalkyl moiety has 3 to 6 carbon atoms, and the alkylene moiety has 1 to 5 carbon atoms; or aralkyl where the aryl moiety has 6 to 10 carbon atoms, and the alkylene moiety has 1 to 5 carbon atoms,
$R^2$ represents a 5- to 7-membered heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or the heterocyclic ring condensed with a benzene ring; or pyridine 1-oxide,
$R^2$ binds to Y via a carbon atom as a ring-constituting atom of $R^2$,
$R^3$ and $R^4$, which are the same or different, represent hydrogen; hydroxy; halogen; cyano; carbamoyl; $C_{1-6}$ alkoxy; $C_{6-10}$ aryloxy; $C_{1-6}$ alkanoyloxy; amino; or acylamino where the acyl moiety has 2 to 6 carbon atoms,
X represents $CH_2$, and
Y represents C(=O),
provided that the $C_{1-6}$ alkyl as $R^1$; the alkylene moiety and cycloalkyl moiety of the cycloalkylalkyl where the cycloalkyl moiety has 3 to 6 carbon atoms, and the alkylene moiety has 1 to 5 carbon atoms as $R^1$; or the alkylene moiety of the aralkyl where the aryl moiety has 6 to 10 carbon atoms, and the alkylene moiety has 1 to 5 carbon atoms as $R^1$ may be substituted with at least one substituent selected from:

1 to 6 halogens; hydroxy; $C_{1-6}$ alkoxy; $C_{6-10}$ aryloxy; $C_{1-6}$ alkanoyl; $C_{1-6}$ alkanoyloxy; carboxyl; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; alkylsulfonyl where the alkyl moiety has 1 to 6 carbon atoms; aminosulfonyl; alkylsulfinyl where the alkyl moiety has 1 to 6 carbon atoms; alkylthio where the alkyl moiety has 1 to 6 carbon atoms; $C_{1-6}$ alkoxy substituted with 1 to 6 halogens; and arylcarbonyl where the aryl moiety has 6 to 10 carbon atoms,
the aryl moiety of the aralkyl where the aryl moiety has 6 to 10 carbon atoms, and the alkylene moiety has 1 to 5 carbon atoms as $R^1$; or the aryl moiety of the $C_{6-10}$ aryloxy as $R^3$ and $R^4$ may be substituted with at least one substituent selected from:

$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy,
the heterocyclic ring as $R^2$ may have, besides the oxo group, the substituents that the aryl moiety of the aralkyl where the aryl moiety has 6 to 10 carbon atoms, and the alkylene moiety has 1 to 5 carbon atoms as $R^1$ mentioned above may have, and pyridine 1-oxide as $R^2$ may have the substituents that the aryl moiety of the aralkyl where the aryl moiety has 6 to 10 carbon atoms, and the alkylene moiety has 1 to 5 carbon atoms as $R^1$ mentioned above may have, and
the alkylene moiety of the aralkyl where the aryl moiety has 6 to 10 carbon atoms, and the alkylene moiety has 1 to 5 carbon atoms as $R^1$ may be substituted with at least one substituent selected from phenyl, and $C_{1-6}$ alkyl substituted with 1 to 3 halogens;

<15> The pharmaceutical composition for treatment or prevention according to <13> or <14>, wherein the compound represented by the general formula (I) is a compound in which $R^1$ represents $C_{1-6}$ alkyl; cycloalkylalkyl where the cycloalkyl moiety has 3 to 6 carbon atoms, and the alkylene moiety has 1 to 5 carbon atoms; or aralkyl where the aryl moiety has 6 to 10 carbon atoms, and the alkylene moiety has 1 to 5 carbon atoms;
<16> The pharmaceutical composition for treatment or prevention according to any one of <13> to <15>, wherein the compound represented by the general formula (I) is a compound in which $R^1$ represents a cycloalkylalkyl where the cycloalkyl moiety has 3 to 6 carbon atoms, and the alkylene moiety has 1 to 5 carbon atoms;
<17> The pharmaceutical composition for treatment or prevention according to <13> or <14>, wherein the compound represented by the general formula (I) is a compound in which $R^1$ represents $C_{2-6}$ alkyl substituted with hydroxy; $C_{1-6}$ alkyl substituted with 1 to 6 halogens; or $C_{2-6}$ alkyl substituted with $C_{1-6}$ alkoxy;
<18> The pharmaceutical composition for treatment or prevention according to <13> or <14>, wherein the compound represented by the general formula (I) is a compound in which $R^1$ represents allyl, fluoropropyl, 2-(pyridin-3-yl)ethyl, 2-(methylsulfonyl)ethyl, or 2-(aminosulfonyl)ethyl;
<19> The pharmaceutical composition for treatment or prevention according to any one of <13> to <18>, wherein the compound represented by the general formula (I) is a compound in which $R^2$ represents a 5- to 7-membered heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or the heterocyclic ring condensed with a benzene ring; or pyridine l-oxide;
<20> The pharmaceutical composition for treatment or prevention according to any one of <13> to <19>, wherein the compound represented by the general formula (I) is a compound in which $R^2$ represents pyridine 1-oxide;
<21> The pharmaceutical composition for treatment or prevention according to any one of <13> to <19>, wherein the compound represented by the general formula (I) is a compound in which the heterocyclic ring as $R^2$ is pyridin-2(1H)-one;
<22> The pharmaceutical composition for treatment or prevention according to any one of <13> to <19>, wherein the compound represented by the general formula (I) is a compound in which the heterocyclic ring as $R^2$ is pyridin-4(1H)-one;
<23> The pharmaceutical composition for treatment or prevention according to any one of <13> to <19>, wherein the compound represented by the general formula (I) is a compound in which the heterocyclic ring as $R^2$ is pyridazin-3(2H)-one;
<24> The pharmaceutical composition for treatment or prevention according to any one of <13> to <19>, wherein the compound represented by the general formula (I) is a compound in which the heterocyclic ring as $R^2$ is pyrazin-2(1H)-one;
<25> The pharmaceutical composition for treatment or prevention according to any one of <13> to <19>, wherein the compound represented by the general formula (I) is a compound in which the heterocyclic ring as $R^2$ is 4H-pyran-4-one or 2H-pyran-2-one;
<26> The pharmaceutical composition for treatment or prevention according to any one of <13> to <19>, wherein the compound represented by the general formula (I) is a compound in which the heterocyclic ring as $R^2$ is quinolin-2(1H)-one;
<27> The pharmaceutical composition for treatment or prevention according to any one of <13> to <19>, wherein the compound represented by the general formula (I) is a compound in which the heterocyclic ring as $R^2$ is pyrimidin-4(3H)-one or pyrimidin-2,4(1H,3H)-dione;
<28> The pharmaceutical composition for treatment or prevention according to any one of <13> and <15> to <27>,

wherein the compound represented by the general formula (1) is a compound in which X represents $CH_2$;
<29> The pharmaceutical composition for treatment or prevention according to any one of <13> to <28>, wherein the compound represented by the general formula (I) is a compound in which one of $R^3$ and $R^4$ represents hydroxy and the other represents hydrogen;
<30> The pharmaceutical composition for treatment or prevention according to any one of <13> and <15> to <28>, wherein the compound represented by the general formula (I) is a compound in which $R^3$ represents halogen; cyano; carbamoyl; $C_{1-6}$ alkoxy; $C_{1-6}$ alkanoyloxy; amino; or acylamino where the acyl moiety has 2 to 6 carbon atoms; $R^4$ represents hydrogen or hydroxy; and $R^5$ represents hydrogen;
<31> The pharmaceutical composition for treatment or prevention according to any one of <13> and <15> to <28>, wherein the compound represented by the general formula (I) is a compound in which $R^3$ represents hydroxy; carbamoyl; or $C_{1-6}$ alkanoyloxy; $R^4$ represents hydrogen; and $R^3$ represents hydrogen;
<32> The pharmaceutical composition for treatment or prevention according to any one of <13> and <15> to <28>, wherein the compound represented by the general formula (I) is a compound in which $R^3$ represents hydroxy; $R^4$ represents hydrogen; and $R^5$ represents hydrogen;
<33> The pharmaceutical composition for treatment or prevention according to any one of <13> and <15> to <28>, wherein the compound represented by the general formula (I) is a compound in which $R^3$, $R^4$ and $R^5$ all represent hydrogen;
<34> The pharmaceutical composition for treatment or prevention according to any one of <13> and <15> to <33>, wherein the compound represented by the general formula (1) is a compound in which $R^{6a}$, $R^{6b}$, $R^7$, $R^8$, $R^9$ and $R^{10}$ all represent hydrogen;
<35> The pharmaceutical composition for treatment or prevention according to any one of <14> to <28>, wherein the compound represented by the general formula (I) is a compound in which $R^3$ represents halogen; cyano; carbamoyl; $C_{1-6}$ alkoxy; $C_{1-6}$ alkanoyloxy; amino; or acylamino where the acyl moiety has 2 to 6 carbon atoms; and $R^4$ represents hydrogen or hydroxy;
<36> The pharmaceutical composition for treatment or prevention according to any one of <14> to <28>, wherein the compound represented by the general formula (I) is a compound in which $R^3$ represents hydroxy; carbamoyl; or $C_{1-6}$ alkanoyloxy; and $R^4$ represents hydrogen;
<37> The pharmaceutical composition for treatment or prevention according to any one of <14> to <28>, wherein the compound represented by the general formula (I) is a compound in which $R^3$ represents hydroxy; and $R^4$ represents hydrogen;
<38> The pharmaceutical composition for treatment or prevention according to any one of <14> to <28>, wherein the compound represented by the general formula (I) is a compound in which $R^3$ and $R^4$ both represent hydrogen;
<39> The pharmaceutical composition for treatment or prevention according to <13>, wherein the compound represented by the general formula (I) is a compound represented by the following general formula (II):

[Formula 8]

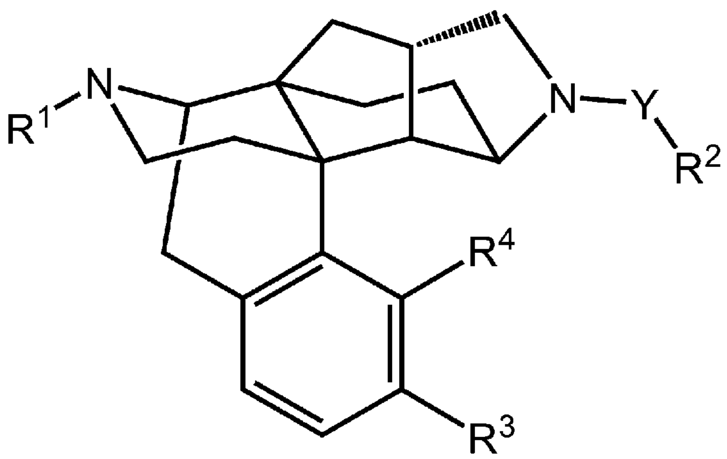

(I I)

(wherein $R^1$ represents hydrogen; $C_{1-6}$ alkyl; or cycloalkylalkyl where the cycloalkyl moiety has 3 to 6 carbon atoms, and the alkylene moiety has 1 to 5 carbon atoms;

$R^2$ represents a 5- to 7-membered heterocyclic ring containing 1 or 2 nitrogen atoms and at least one carbon

atom as ring-constituting atoms, containing at least one set of adjacent ring-constituting atoms bound by a double bond, and further substituted with at least one oxo group; or pyridine 1-oxide;
$R^2$ binds to Y via a carbon atom as a ring-constituting atom of $R^2$,
$R^3$ and $R^4$, which are the same or different, represent hydrogen; hydroxy; or $C_{1-6}$ alkoxy,
Y represents C(=O),
provided that the $C_{1-6}$ alkyl as $R^1$; and the alkylene moiety and cycloalkyl moiety of the cycloalkylalkyl where the cycloalkyl moiety has 3 to 6 carbon atoms and the alkylene moiety has 1 to 5 carbon atoms as $R^1$ may be substituted with at least one substituent selected from:

1 to 6 halogens; hydroxy; and $C_{1-6}$ alkoxy,
the heterocyclic ring as $R^2$ may have, besides the oxo group, at least one substituent selected from $C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy, and
the pyridine 1-oxide as $R^2$ may have at least one substituent selected from:
$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy);

<40> The pharmaceutical composition for treatment or prevention according to <39>, wherein the compound represented by the general formula (II) is a compound in which $R^1$ is cycloalkylalkyl where the cycloalkyl moiety has 3 to 6 carbon atoms, and the alkylene moiety has 1 to 5 carbon atoms;
<41> The pharmaceutical composition for treatment or prevention according to <39> or <40>, wherein the compound represented by the general formula (II) is a compound in which $R^2$ is represented by a formula (III):

[Formula 9]

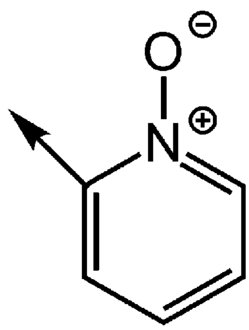

(III)

a formula (IV):

[Formula 10]

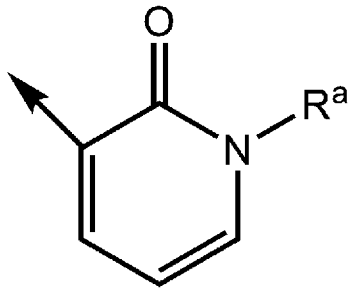

(IV)

a formula (V):

[Formula 11]

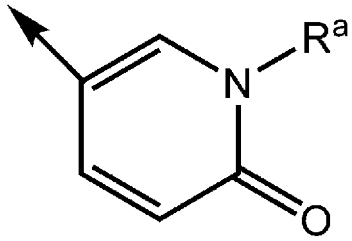

(V)

or a formula (VI):

[Formula 12]

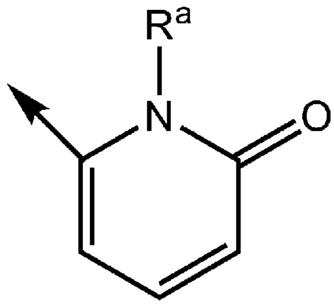

(VI)

(wherein in formulas (III) to (VI), $R^a$ represents hydrogen or $C_{1-6}$ alkyl, and binding to Y occurs at the position of the arrow);

<42> The pharmaceutical composition for treatment or prevention according to any one of <39> to <41>, wherein the compound represented by the general formula (II) is a compound in which $R^3$ is hydroxy;

<43> The pharmaceutical composition for treatment or prevention according to any one of <39> to <42>, wherein the compound represented by the general formula (II) is a compound in which $R^4$ is hydrogen;

<44> The pharmaceutical composition for treatment or prevention according to <13>, wherein the compound represented by the general formula (I) is at least one compound selected from:

2-((1S,3aR,5aS,6R,11bR,11cS)-14-(cyclopropylmethyl)-10-hydroxy-2,3,3a,4,5,6,7,11c-octahydro-1H-6,1b-(epiminoethano)-1,5a-methanonaphtho[1,2-e]indole-3-carbonyl)pyridine 1-oxide,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
3-((1S,3aR,5aS,6R,11bR,11cS)-14-(cyclopropylmethyl)-10-hydroxy-2,3,3a,4,5,6,7,11c-octahydro-1H-6,1b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one.
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-6-methylpyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidine-2,4(1H,3H)-dione,
3-((1S,3aR,5aS,6R,11bR,11cS)-14-(cyclopropylmethyl)-10-hydroxy-2,3,3a,4,5,6,7,11c-octahydro-1H-6,11b-(epiminethano)-1,5a-methanonaphtho[1,2-e]indole-3-carbonyl)pyridin-4(1H)-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-4(1H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridazin-3(2H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)quinolin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-2H-pyran-2-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-4H-pyran-4-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-4(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrazin-2(1H)-one,
2-((1S,3aR,5aS,6R,11bR,11cS)-10-acetoxy-14-(cyclopropylmethyl)-2,3,3a,4,5,6,7,11c-octahydro-1H-6,11b-(epiminoethano)-1,5a-methanonaphtho[1,2-e]indole-3-carbonyl)pyridine 1-oxide,
6-((1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrazin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidine-2,4(1H,3H)-dione,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidin-4(3H)-one, and
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one;

<45> The pharmaceutical composition for treatment or prevention according to any one of <13> and <39> to <43>, wherein the compound represented by the general formula (I) is at least one compound selected from:

2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one, and
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one.

[Effects of Invention]

The present invention provides a pharmaceutical composition that is useful for the treatment or prevention of stress-related disorders, stress-induced anxiety disorders, or stress-related disorders or anxiety disorders associated with depression.

[Brief Description of Drawings]

FIG. 1 is a diagram illustrating the results of tests relating to the mechanism of action for emotion regulation for the compound of Example 7.
FIG. 2 is a diagram illustrating the effects on the reconsolidation of fear memories for the compound of Example 7.
FIG. 3 is a diagram illustrating the effects on the reconsolidation of fear memories for the compound of Example 7.

[Description of Embodiments]

The present invention is described below in further detail.

In this description:
the term "δ-opioid receptor agonist" means a substance which, by binding with the δ-opioid receptor, induces a physiological response reaction the same as, or similar to, an endogenous ligand.

The term "selective δ-opioid receptor agonist" means a substance for which the δ-opioid receptor agonist action is relatively strong compared with the μ-opioid receptor agonist action and the κ-opioid receptor agonist action. Among these substances, those having a δ-opioid receptor agonist action, and either having only extremely weak μ-opioid receptor agonist action and κ-opioid receptor agonist action, or not having these actions at all, are preferred. Moreover, substances having a δ-opioid receptor agonist action, having no μ-opioid receptor agonist action nor κ-opioid receptor agonist action, and also having a κ-opioid receptor antagonist action are preferred. In addition, substances having a δ-opioid receptor agonist action, having no μ-opioid receptor agonist action nor κ-opioid receptor agonist action, and also having a μ-opioid receptor antagonist action and a κ-opioid receptor antagonist action are preferred. Here, the term "μ-opioid receptor antagonist" or "κ-opioid receptor antagonist" means a substance that binds to the μ-opioid receptor or x-opioid receptor, but unlike an endogenous ligand, does not induce a physiological response reaction, but rather, as a result of the binding, inhibits binding between the receptor and the endogenous ligand that would naturally bind, thereby suppressing the physiological response reaction of the endogenous ligand.

The expression "stress-related disorder" means a mental disorder that develops mainly due to acute or chronic psychological stress, and examples include acute stress disorder, post-traumatic stress disorder, and adjustment disorder. The symptoms and diagnostic criteria for "acute stress disorder (also called acute stress reaction)", "post-traumatic stress disorder", or "adjustment disorder" may follow those described in "The Diagnostic and Statistical Manual of Mental Disorders, Fifth Edition" (sometimes abbreviated as DSM-5) published by the American Psychiatric Association. Accordingly, for example, post-traumatic stress disorder (sometimes abbreviated as PTSD), can be diagnosed by reference to the following items A to H.

A. Exposure to actual or threatened death, serious injury, or sexual violence in one (or more) of the following ways:

(1) Directly experiencing the traumatic event(s).
(2) Witnessing, in person, the event(s) as it occurred to others.
(3) Learning that the traumatic event(s) occurred to a close family member or close friend. In cases of actual or threatened death of a family member or friend, the event(s) must have been violent or accidental.
(4) Experiencing repeated or extreme exposure to aversive details of the traumatic event(s) (for example, first

responders collecting human remains, police officers repeatedly exposed to details of child abuse).

B. Presence of one (or more) of the following intrusion symptoms associated with the traumatic event(s), beginning after the traumatic event(s) occurred.

(1) Recurrent, involuntary, and intrusive distressing memories of the traumatic event(s).
(2) Recurrent distressing dreams in which the content and/or emotions of the dream are related to the traumatic event(s).
(3) Dissociative symptoms (for example, flashbacks) causing feelings or actions as if the traumatic event were recurring (such reactions may occur on a continuum, with the most extreme expression being a complete loss of awareness of present surroundings).
(4) Intense or prolonged psychological distress at exposure to internal or external cues that symbolize or resemble an aspect of the traumatic event(s).
(5) Marked physiological reactions to internal or external cues that symbolize or resemble an aspect of the traumatic event(s).

C. Persistent avoidance of stimuli associated with the traumatic event(s), beginning after the traumatic event(s) occurred, as evidenced by one or both of the following.

(1) Avoidance of or efforts to avoid distressing memories, thoughts, or feelings about or closely associated with the traumatic event(s).
(2) Avoidance of or efforts to avoid external reminders (people, places, conversations, activities, objects, situations) that arouse distressing memories, thoughts, or feelings about or closely associated with the traumatic event(s).

D. Negative alterations in cognitions and mood associated with the traumatic event(s), beginning or worsening after the traumatic event(s) occurred, as evidenced by two (or more) of the following.

(1) Inability to remember an important aspect of the traumatic event(s) (typically due to dissociative amnesia and not to other factors such as head injury, alcohol, or drugs).
(2) Persistent and exaggerated negative beliefs or expectations about oneself, others, or the world (for example, "I am bad", "No one can be trusted", "The world is completely dangerous", "My whole nervous system is permanently ruined").
(3) Persistent, distorted cognitions about the cause or consequences of the traumatic event(s) that lead the individual to blame himself/herself or others.
(4) Persistent negative emotional state (for example, fear, horror, anger, guilt, or shame).
(5) Markedly diminished interest or participation in significant activities.
(6) Feelings of detachment or estrangement from others.
(7) Persistent inability to experience positive emotions (for example, inability to experience happiness, satisfaction, or loving feelings).

E. Marked alterations in arousal and reactivity associated with the traumatic event(s), beginning or worsening after the traumatic event(s) occurred, as evidenced by two (or more) of the following.

(1) Irritable behavior and angry outbursts (with little or no provocation) typically expressed as verbal or physical aggression toward people or objects.
(2) Reckless or self-destructive behavior.
(3) Hypervigilance.
(4) Exaggerated startle response.
(5) Problems with concentration.
(6) Sleep disturbance for example, difficulty falling or staying asleep or restless sleep).

F. Duration of the disorder (Criteria B, C, D, and E) is 1 month or longer.
G. The disorder causes clinically significant distress or impairment in social, occupational, or other important areas of functioning.
H. The disorder is not attributable to the physiological effects of a substance (for example, medication or alcohol) or another medical condition.

Acute stress disorder is a transient disturbance, and although the clinical symptoms are basically the same as those of post-traumatic stress disorder, they do not persist for longer than 1 month.

For example, in order to diagnose acute stress disorder, an individual must have been exposed either directly or indirectly to a traumatic event, and exhibit at least 9 of the following symptoms from 1) to 14) for at least 3 days and up to a month.

1) Recurrent, involuntary, and intrusive distressing memories associated with the traumatic event(s)
2) Recurrent distressing dreams associated with the traumatic event(s)
3) Dissociative reactions (for example, flashbacks) causing feelings as if the traumatic event were recurring
4) Intense or psychological or physiological distress when remembering the event(s) (for example, on the anniversary of the event(s), distress caused by a sound similar to that heard during the event(s))
5) Persistent inability to experience positive emotions (for example, inability to experience happiness, satisfaction, or loving feelings)
6) An altered sense of reality (for example, being in a daze, time slowing, alteration in perspective of events)
7) Inability to remember an important aspect of the traumatic event(s)
8) Efforts to avoid distressing memories, thoughts, or feelings associated with the traumatic event(s)
9) Efforts to avoid external reminders (people, places, conversations, activities, objects, situations) that arouse memories associated with the traumatic event(s)
10) Sleep disturbance
11) Irritable behavior and angry outbursts
12) Hypervigilance
13) Problems with concentration
14) Exaggerated startle response

Moreover, the symptoms must cause marked distress, or markedly impair social or occupational functions, and must not be due to the physiological effects of a substance or another physical medical condition.

Adjustment disorder is a disturbance in which an individual is unable to cope with various stresses that occur during life, resulting in the development of mental symptoms such as depression or anxiety that impact daily life.

For example, in order to diagnose adjustment disorder, an individual must exhibit all of the following symptoms A to E.

A. The development of symptoms in response to an readily identifiable stressor(s) occurring within 3 months of the onset of the stressor(s).

B. These symptoms are evidenced by at least one of the following:

- Symptoms and/or distress that is out of proportion to the stressor
- Significant impairment in important areas of life function, such as social and occupational areas

C. The symptoms cannot be explained by another mental disorder

D. The symptoms do not represent a normal bereavement reaction

E. Once the stressor or its consequences has terminated, the symptoms do not persist beyond 6 months.

The pharmaceutical composition according to the present invention is useful in the suppression of the symptoms described above in B.

The pharmaceutical composition according to the present invention can suppress flashbacks. For example, by administering the pharmaceutical composition according to the present invention to patients suffering from acute stress disorder or post-traumatic stress disorder, flashbacks are suppressed.

The pharmaceutical composition according to the present invention can suppress avoidance of events or matters associated with a trauma. For example, by administering the pharmaceutical composition according to the present invention to patients suffering from acute stress disorder or post-traumatic stress disorder, the tendency for the patient to avoid events or matters associated with the trauma is suppressed.

One aspect of the present invention is the pharmaceutical composition for treatment or prevention described above, the composition having a facilitation effect on the extinction of memories of a traumatic event, and/or an inhibitory effect on the reconsolidation of memories of a traumatic event. Here, the expression "reconsolidation of memories of a traumatic event" means a process, similar to extinction learning, wherein re-exposure to the traumatic event(s) results in stabilization or intensification of previously formed memories of the traumatic event(s).

In this description, "suppression" is a concept that includes halting or slowing the worsening or progression of symptoms, a condition or a disorder, and actions or measures for achieving the same, as well as ameliorating the above

symptoms, condition or disorder, and actions or measures for achieving the same. The expression "worsening or progression of symptoms, a condition or a disorder" includes the worsening or progression of "pathological" or "abnormal" symptoms, conditions or disorders, and the worsening or progression from a "healthy" or "normal" state to a state of "pathological" or "abnormal" symptoms, conditions or disorders. In one embodiment, "suppression" means halting or slowing the worsening or progression of symptoms, a condition or a disorder, and actions or measures for achieving the same. In another embodiment, "suppression" means halting or slowing the worsening or progression of symptoms, a condition or a disorder. The term "amelioration" is a concept that includes shifting a "pathological" or "abnormal" symptom, condition or disorder closer to a "healthy" or "normal" state, or an action or measure for achieving the same, as well as replacing a "pathological" or "abnormal" symptom, condition or disorder with a "healthy" or "normal" state, or an action or measure for achieving the same. Accordingly, in one embodiment, "amelioration" includes the movement of a numerical value that acts as an indicator of a "pathological" or "abnormal" symptom, condition or disorder to either a smaller value or larger value that is closer to a normal value, or to a value that is a normal value, in accordance with the "amelioration". In this description, the term "palliation" may be a concept that includes the above "amelioration".

In this description, the term "treatment" is a concept that includes eliminating, curing, healing or achieving remission of a "pathological" or "abnormal" symptom, condition or disorder, and actions or measures for achieving the same, as well as including "suppression" of the worsening of a "pathological" or "abnormal" symptom, condition or disorder, and actions or measures for achieving the same, and "amelioration". In one embodiment, "treatment" means eliminating, curing, healing or achieving remission of a "pathological" or "abnormal" symptom, condition or disorder, as well as an action or measure for achieving the same. In another embodiment, "treatment" means eliminating, curing, healing or achieving remission of a "pathological" or "abnormal" symptom, condition or disorder.

In this description, the term "prevention" is a concept that includes preventing the onset of a "pathological" or "abnormal" symptom, condition or disorder, and actions or measures for achieving the same.

In this description:
examples of the $C_{1-6}$ alkyl include methyl, ethyl, propyl, i-propyl, butyl, tert-butyl, pentyl, neopentyl and hexyl.

Examples of the $C_{1-10}$ alkyl include the $C_{1-6}$ alkyl groups mentioned above, as well as heptyl and octyl.

Examples of the $C_{1-6}$ alkyl substituted with 1 to 3 halogens include 2-chloroethyl, 2-fluoroethyl, 3-fluoropopyl, 2,2-difluoroethyl, trifluoromethyl, and 3,3,3-trifluoropropyl .

Examples of the $C_{2-6}$ alkenyl include 2-propenyl and 3-methyl-2-butenyl.

Examples of the cycloalkylalkyl where the cycloalkyl moiety has 3 to 6 carbon atoms and the alkylene moiety has 1 to 5 carbon atoms include methyl or ethyl or the like substituted with $C_{3-6}$ cycloalkyl such as cyclopropyl, cyclobutyl, cyclopentyl or cyclohexyl.

Examples of the aralkyl where the aryl moiety has 6 to 10 carbon atoms and the alkylene moiety has 1 to 5 carbon atoms include a benzyl group and phenethyl group.

Examples of the $C_{3-6}$ cycloalkyl include cyclopropyl, cyclobutyl, cyclopentyl and cyclohexyl.

Examples of the $C_{6-10}$ aryl include phenyl and naphthyl.

Examples of the heteroaryl containing 1 to 4 heteroatoms selected from N, O and S as ring-constituting atoms include pyridyl, furyl, imidazolyl, pyrazolyl, pyrimidinyl, pyrazinyl, pyridazinyl and thiazolyl.

Examples of the heteroarylalkyl where the heteroaryl contains 1 to 4 heteroatoms selected from N, O and S as ring-constituting atoms, and the alkylene moiety has 1 to 5 carbon atoms include (pyridin-2-yl)methyl, (pyridin-3-yl)methyl, (pyridin-4-yl)methyl, (furan-2-yl)methyl, (furan-3-yl)methyl, (imidazol-2-yl)methyl, (imidazol-4-yl)methyl, (imidazol-5-yl)methyl, (thiazol-2-yl)methyl, (thiazol-4-yl)methyl, (thiazol-5-yl)methyl, 2-(pyridin-2-yl)ethyl, 2-(pyridin-3-yl)ethyl, 2-(pyrazol-1-yl)ethyl, 2-(thiophen-2-yl)ethyl, and 2-(thiophen-3-yl)ethyl.

Examples of the $C_{1-6}$ alkanoyl include acetyl and propionyl.

Examples of the $C_{1-6}$ alkoxy include methoxy, ethoxy and propoxy.

Examples of the $C_{1-6}$ alkanoyloxy include acetoxy and the like.

Examples of the alkoxycarbonyl where the alkoxy moiety has 1 to 6 carbon atoms include methoxycarbonyl and ethoxycarbonyl.

Examples of the halogens include fluorine, chlorine, bromine and iodine.

Examples of the $C_{1-6}$ alkoxy substituted with 1 to 3 halogens include fluoromethoxy and trifluoromethoxy.

Examples of the $C_{1-6}$ alkoxy substituted with 1 to 6 halogens include the $C_{1-6}$ alkoxy substituted with 1 to 3 halogens mentioned above, as well as tetrafluoroethoxy and the like.

Examples of the phenylalkyl where the alkyl has 1 to 3 carbon atoms include benzyl and the like.

Examples of the $C_{6-10}$ aryloxy include phenoxy and the like.

Examples of the $C_{1-8}$ alkylamino include methylamino and ethylamino.

Examples of the acylamino where the acyl moiety has 2 to 6 carbon atoms include acetylamino and the like.

Examples of the $C_{6-10}$ arylamino include phenylamino and the like.

Examples of the alkylcarbamoyl where the alkyl moiety has 1 to 6 carbon atoms include ethylcarbamoyl and the like.

Examples of the dialkylcarbamoyl where each alkyl moiety has 1 to 6 carbon atoms include diethylcarbamoyl and the like.

Examples of the alkylsulfonyl where the alkyl moiety has 1 to 6 carbon atoms include methylsulfonyl and the like.

Examples of the alkylsulfinyl where the alkyl moiety has 1 to 6 carbon atoms include methylsulfinyl and the like.

Examples of the alkylthio where the alkyl moiety has 1 to 6 carbon atoms include methylthio and the like.

Examples of the arylcarbonyl where the aryl moiety has 6 to 10 carbon atoms include benzoyl and the like.

Examples of the 5- to 7-membered ring that may be formed from $R^{11}$ and $R^{12}$, the nitrogen atom to which $R^{11}$ and $R^{12}$ are bonded, and optionally 1 or 2 other heteroatoms include pyrrolidine, piperidine and morpholine.

Examples of the heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group, or the pyridine 1-oxide, as $R^2$ include:

(A) pyridine 1-oxide which may be substituted with 1 to 4 substituents selected from $C_{1-10}$ alkyl substituted with 1 to 3 fluorine atoms and unsubstituted $C_{1-10}$ alkyl, such as pyridine 1-oxide and 2-methylpyridine 1-oxide,
(B) pyridin-2(1H)-one which may be substituted with 1 to 4 substituents selected from $C_{1-10}$ alkyl substituted with 1 to 3 fluorine atoms and unsubstituted $C_{1-10}$ alkyl, such as pyridin-2(1H)-one, 1-methylpyridin-2(1H)-one, 1-ethylpyridin-2(1H)-one, 6-methylpyridin-2(1H)-one, 6-ethylpyridin-2(1H)-one, and 6-trifluoromethylpyridin-2(1H)-one,
(C) pyridin-4(1H)-one which may be substituted with 1 to 4 substituents selected from $C_{1-10}$ alkyl substituted with 1 to 3 fluorine atoms and unsubstituted $C_{1-10}$ alkyl, such as pyridin-4(1H)-one, 1-methylpyridin-4(1H)-one, 1-ethylpyridin-4(1H)-one, and 1-(fluoroethyl)pyridin-4(1H)-one,
(D) pyridazin-3(2H)-one which may be substituted with 1 to 3 substituents selected from $C_{1-10}$ alkyl substituted with 1 to 3 fluorine atoms and unsubstituted $C_{1-10}$ alkyl, such as pyridazin-3(2H)-one and 2-methylpyridazin-3(2H)-one,
(E) pyrazin-2-(1H)-one which may be substituted with 1 to 3 substituents selected from $C_{1-10}$ alkyl substituted with 1 to 3 fluorine atoms and unsubstituted $C_{1-10}$ alkyl, such as pyrazin-2-(1H)-one and 1-methylpyrazin-2-(1H)-one,
(F) 4H-pyran-4-one or 2H-pyran-2-one which may be substituted with 1 to 3 substituents selected from $C_{1-10}$ alkyl substituted with 1 to 3 fluorine atoms and unsubstituted $C_{1-10}$ alkyl, such as 4H-pyran-4-one, 3-methyl-4H-pyran-4-one, 2H-pyran-2-one, and 5-methyl-2H-pyran-2-one,
(G) quinolin-2(1H)-one or quinoline 1-oxide which may be substituted with 1 to 3 substituents selected from $C_{1-10}$ alkyl substituted with 1 to 3 fluorine atoms and unsubstituted $C_{1-10}$ alkyl, such as quinolin-2(1H)-one, 6-methylquinolin-2(1H)-one, quinoline 1-oxide, and 4-methylquinoline 1-oxide,
(H) pyrimidin-4(3H)-one or pyrimidin-2,4(1H,3H)-dione which may be substituted with 1 to 3 substituents selected from $C_{1-10}$ alkyl substituted with 1 to 3 fluorine atoms and unsubstituted $C_{1-10}$ alkyl, such as pyrimidin-4(3H)-one and pyrimidin-2,4(1H,3H)-dione.

Examples of tautomers of the compound represented by the general formula (I) include tautomers of the aforementioned heterocyclic ring as $R^2$, containing 1 to 4 heteroatoms selected from N, O and S and at least one carbon atom as ring-constituting atoms, containing at least one set of adjacent ring-constituting atoms bound by a double bond, and further substituted with at least one oxo group, and an example is 2-hydroxypyridine (lactim) that corresponds with 2-pyridone (lactam).

Among the compounds represented by the above general formula (I), tautomers and stereoisomers of those compounds, pharmaceutically acceptable salts thereof, or solvates of any of the above, examples of preferred pharmaceutically acceptable salts include acid addition salts, wherein specific examples of those acid addition salts include salts with organic acids or inorganic acids such as hydrochloride, sulfate, fumarate, oxalate, methanesulfonate and camphorsulfonate.

Among the compounds represented by the above general formula (I), tautomers and stereoisomers of those compounds, and pharmaceutically acceptable salts thereof, or solvates of any of the above, examples of the stereoisomers include cis and trans isomers, racemates, and optically active compounds.

Among the compounds represented by the above general formula (I), tautomers and stereoisomers of those compounds, and pharmaceutically acceptable salts thereof, or solvates of any of the above, examples of the solvates include pharmaceutically acceptable solvates of the compound of the present invention or a salt thereof, and also include hydrates.

Further, the compound represented by the above general formula (I), a tautomer or stereoisomer of that compound, a pharmaceutically acceptable salt thereof, or a solvate thereof may be a prodrug that has been chemically modified so as to change into the pharmacologically active substance in vivo or upon reaching the target site, thereby realizing the pharmacological effect (activation).

Examples of the prodrug, for example in those cases where the group that constitutes the prodrug exists at a hydroxy group, include typical hydroxy group protective groups such as lower acyl groups and lower alkoxycarbonyl groups, in those cases where the group that constitutes the prodrug exists at a nitrogen atom, include typical amino

group protective group such as lower acyl groups and lower alkoxycarbonyl groups, or in those cases where the prodrug group is introduced at a carboxylic acid site, include a pivaloyloxymethyl (tBu-C(O)O-$CH_2$-) group, a Medoxomil group, and a Cilexetil group.

Moreover, the compound represented by the above general formula (I), a tautomer or stereoisomer of that compound, a pharmaceutically acceptable salt thereof, or a solvate of any of the above may be substituted with a stable isotope such as deuterium.

The compound represented by the above general formula (I), a tautomer or stereoisomer of that compound, a pharmaceutically acceptable salt thereof, or a solvate of any of the above can be produced, for example, in accordance with the method described in WO2018/052114 or conventional methods.

The compound represented by the above general formula (I), a tautomer or stereoisomer of that compound, a pharmaceutically acceptable salt thereof, or a solvate of any of the above exhibits superior agonist activity and selectivity for the δ-opioid receptor compared with the μ- and κ-opioid receptors.

Accordingly, the compound represented by the above general formula (1), a tautomer or stereoisomer of that compound, a pharmaceutically acceptable salt thereof, or a solvate of any of the above can be used in a pharmaceutical composition that exhibits a selective δ-opioid receptor agonist action.

Moreover, the compound represented by the above general formula (I), a tautomer or stereoisomer of that compound, a pharmaceutically acceptable salt thereof, or a solvate of any of the above exhibits excellent stability relative to metabolism by human liver microsome.

Accordingly, the compound represented by the above general formula (I), a tautomer or stereoisomer of that compound, a pharmaceutically acceptable salt thereof, or a solvate of any of the above can be used in an orally administered pharmaceutical composition.

The pharmaceutical composition provided by the present invention may be administered either orally or parenterally to humans or other mammals, wherein examples of the parenteral administration include intravenous administration, subcutaneous administration, intramuscular administration, intra-articular administration, transmucosal administration, percutaneous administration, transnasal administration, rectal administration, and intrathecal administration.

The pharmaceutical composition provided by the present invention may be prepared by using the selective δ-opioid receptor agonist described above, or the compound represented by the above general formula (1), a tautomer or stereoisomer of that compound, a pharmaceutically acceptable salt thereof, or a solvate of any of the above, either as is, or in a mixture with one or more pharmaceutically acceptable carriers, such as excipients (for example, lactose, D-mannitol, crystalline cellulose and glucose), binders (for example, hydroxypropylcellulose (HPC), gelatin, and polyvinylpyrrolidone (PVP)), lubricants (for example, magnesium stearate and talc), disintegrants (for example, starch, and carboxymethylcellulose calcium (CMC-Ca)), diluents (for example, injection water and physiological saline solution), and other additives as necessary (for example, pH modifiers, surfactants, solubilizers, preservatives, emulsifiers, tonicity agents, and stabilizers), and may be prepared in various formulations, including tablets, granules, powders, capsules, suspensions, injections, and suppositories. For example, in order to prepare a tablet, the compound represented by the above general formula (I), a tautomer or stereoisomer of that compound, a pharmaceutically acceptable salt thereof, or a solvate of any of the above may be mixed and formulated with an excipient (such as lactose, D-mannitol, crystalline cellulose or glucose), a disintegrant (such as starch or carboxymethylcellulose calcium (CMC-Ca)), a binder (such as hydroxypropylcellulose (HPC), gelatin or polyvinylpyrrolidone (PVP)), and a lubricant (such as magnesium stearate or talc) and the like. In order to prepare an injection, the compound represented by the above general formula (I), a tautomer or stereoisomer of that compound, a pharmaceutically acceptable salt thereof, or a solvate of any of the above may be mixed and formulated with a dispersant (for example, a surfactant such as Tween 80, a polysaccharide such as carboxymethylcellulose, sodium alginate or hyaluronic acid, or polysorbate), a preservative (such as methylparaben or propylparaben), a tonicity agent (such as sodium chloride, mannitol, sorbitol or glucose), and a pH modifier (such as sodium phosphate or potassium phosphate) and the like.

The pharmaceutical composition provided by the present invention may contain the compound represented by the above general formula (I), a tautomer or stereoisomer of that compound, a pharmaceutically acceptable salt thereof, or a solvate of any of the above in an amount that is effective in the treatment or prevention of a stress-related disorder, a stress-induced anxiety disorder, or a stress-related disorder or anxiety disorder associated with depression.

In one embodiment, the pharmaceutical composition provided by the present invention may contain the compound represented by the above general formula (1), a pharmaceutically acceptable solvate (such as a hydrate) of that compound, a pharmaceutically acceptable salt of the compound, or a pharmaceutically acceptable solvate (such as a hydrate) of that salt, in an amount that is effective in the treatment or prevention of a stress-related disorder, a stress-induced anxiety disorder, or a stress-related disorder or anxiety disorder associated with depression.

In one embodiment, the pharmaceutical composition provided by the present invention may contain the compound represented by the above general formula (1), or a pharmaceutically acceptable salt of the compound, in an amount that is effective in the treatment or prevention of a stress-related disorder, a stress-induced anxiety disorder, or a stress-related disorder or anxiety disorder associated with depression.

The dosage of the compound represented by the above general formula (I), tautomer or stereoisomer of that compound, pharmaceutically acceptable salt thereof, or solvate of any of the above may be determined appropriately in accordance with factors such as the type of salt, the administration method, and the symptoms and age of the administration subject. For example, in those cases where the compound represented by the above general formula (I), a tautomer or stereoisomer of that compound, a pharmaceutically acceptable salt thereof, or a solvate of any of the above is administered orally to a human, the dosage is typically within a range from 1 μg/day to 10 g/day, preferably from 0.01 to 2,000 mg/day, more preferably from 0.1 to 100 mg/day, whereas in the case of intravenous administration to a human, the dosage is typically within a range from 0.1 μg/day to 1 g/day, and preferably from 0.001 to 200 mg/day. Administration may be split across 1 to 3 doses per day.

Further, the present invention also has the following aspects.

<1a> A method for the treatment or prevention of stress-related disorders, stress-induced anxiety disorders, or stress-related disorders or anxiety disorders associated with depression, the method including administering a therapeutically effective amount of a selective δ-opioid receptor agonist to a subject (for example, a mammal such as a human) requiring the treatment or prevention;
<2a> The method according to <1a>, wherein the selective δ-opioid receptor agonist also has a κ-opioid receptor antagonist action;
<3a> The method according to <1a> or <2a>, wherein the selective δ-opioid receptor agonist also has a μ-opioid receptor antagonist action and a κ-opioid receptor antagonist action;
<4a> The method according to any one of <1a> to <3a>, wherein the stress-related disorder is an acute stress disorder, post-traumatic stress disorder, or adjustment disorder;
<5a> The method according to any one of <1a> to <4a>, wherein the stress-related disorder is an acute stress disorder or post-traumatic stress disorder;
<6a> The method according to <4a> or <5a> which is a method for the treatment or prevention of post-traumatic stress disorder, the method including suppressing intrusion symptoms related to the traumatic event, suppressing persistent avoidance of stimuli associated with the traumatic event, or suppressing negative changes in cognitions and mood related to the traumatic event, that begin following the traumatic event;
<7a> The method according to <6a>, wherein the intrusion symptoms related to the traumatic event that begin following the traumatic event include at least one symptom among (1) to (5) described below:

(1) recurrent, involuntary and intrusive painful recollections of the traumatic event;
(2) recurrent distressing dreams in which dream content and/or emotions are related to the traumatic event;
(3) dissociative symptoms causing feelings or actions of the traumatic event reoccurring;
(4) intense or prolonged psychological pain upon exposure to internal or external cues that symbolize aspects of the traumatic event or something similar; and
(5) clear physiological reactions to internal or external cues that symbolize aspects of the traumatic event or something similar;

<8a> The method according to any one of <1a> to <7a>, wherein the method suppresses flashbacks;
<9a> The method according to any one of <1a> to <8a>, wherein the method suppresses avoidance of events or matters related to a trauma;
<10a> The method according to any one of <1a> to <9a>, wherein the method facilitates the extinction of memories of a traumatic event, and/or inhibits the reconsolidation of memories of a traumatic event;
<11a> The method according to <10a>, wherein the facilitation of the extinction of memories of a traumatic event is a facilitation of extinction learning of memories of a traumatic event;
<12a> The method according to any one of <1a> to <11a>, wherein the method palliates fear from fear memories;
<1b> A selective δ-opioid receptor agonist for use in the treatment or prevention of stress-related disorders, stress-induced anxiety disorders, or stress-related disorders or anxiety disorders associated with depression;
<2b> The selective δ-opioid receptor agonist for use according to <1b>, wherein the selective δ-opioid receptor agonist also has a x-opioid receptor antagonist action;
<3b> The selective δ-opioid receptor agonist for use according to <1b> or <2b>, wherein the selective δ-opioid receptor agonist also has a μ-opioid receptor antagonist action and a x-opioid receptor antagonist action;
<4b> The selective δ-opioid receptor agonist for use according to any one of <1b> to <3b>, wherein the stress-related disorder is an acute stress disorder, post-traumatic stress disorder, or adjustment disorder;
<5b> The selective δ-opioid receptor agonist for use according to any one of <1b> to <4b>, wherein the stress-related disorder is an acute stress disorder or post-traumatic stress disorder;
<6b> The selective δ-opioid receptor agonist for use according to <4b> or <5b> which is used for the treatment or prevention of post-traumatic stress disorder, which includes suppressing intrusion symptoms related to the traumatic

event, suppressing persistent avoidance of stimuli associated with the traumatic event, or suppressing negative changes in cognitions and mood related to the traumatic event, that begin following the traumatic event;
<7b> The selective δ-opioid receptor agonist for use according to <6b>, wherein the intrusion symptoms related to the traumatic event that begin following the traumatic event include at least one symptom among (1) to (5) described below:

(1) recurrent, involuntary and intrusive painful recollections of the traumatic event;
(2) recurrent distressing dreams in which dream content and/or emotions are related to the traumatic event;
(3) dissociative symptoms causing feelings or actions of the traumatic event reoccurring;
(4) intense or prolonged psychological pain upon exposure to internal or external cues that symbolize aspects of the traumatic event or something similar; and
(5) clear physiological reactions to internal or external cues that symbolize aspects of the traumatic event or something similar;

<8b> The selective δ-opioid receptor agonist for use according to any one of <1b> to <7b>, wherein the selective δ-opioid receptor agonist suppresses flashbacks;
<9b> The selective δ-opioid receptor agonist for use according to any one of <1b> to <8b>, wherein the selective δ-opioid receptor agonist suppresses avoidance of events or matters related to a trauma;
<10b> The selective δ-opioid receptor agonist for use according to any one of <1b> to <9b>, wherein the selective δ-opioid receptor agonist has a facilitation effect on the extinction of memories of a traumatic event, and/or an inhibitory effect on the reconsolidation of memories of a traumatic event;
<11b> The selective δ-opioid receptor agonist for use according to <10b>, wherein the facilitation effect on the extinction of memories of a traumatic event is a facilitation effect on extinction learning of memories of a traumatic event;
<12b> The selective δ-opioid receptor agonist for use according to any one of <1b> to <11b>, wherein the selective δ-opioid receptor agonist palliates fear from fear memories;
<1c> Use of a selective δ-opioid receptor agonist for producing a pharmaceutical composition for the treatment or prevention of stress-related disorders, stress-induced anxiety disorders, or stress-related disorders or anxiety disorders associated with depression;
<2c> The use according to <1c>, wherein the selective δ-opioid receptor agonist also has a κ-opioid receptor antagonist action;
<3c> The use according to <1c> or <2c>, wherein the selective δ-opioid receptor agonist also has a μ-opioid receptor antagonist action and a κ-opioid receptor antagonist action;
<4c> The use according to any one of <1c> to <3c>, wherein the stress-related disorder is an acute stress disorder, post-traumatic stress disorder, or adjustment disorder;
<5c> The use according to any one of <1c> to <4c>, wherein the stress-related disorder is an acute stress disorder or post-traumatic stress disorder;
<6c> The use according to <4c> or <5c> for producing a pharmaceutical composition for the treatment or prevention of post-traumatic stress disorder that has an inhibitory effect on intrusion symptoms related to the traumatic event, an inhibitory effect on persistent avoidance of stimuli associated with the traumatic event, or an inhibitory effect on negative changes in cognitions and mood related to the traumatic event, that begin following the traumatic event;
<7c> The use according to <6c>, wherein the intrusion symptoms related to the traumatic event that begin following the traumatic event include at least one symptom among (1) to (5) described below:

(1) recurrent, involuntary and intrusive painful recollections of the traumatic event;
(2) recurrent distressing dreams in which dream content and/or emotions are related to the traumatic event;
(3) dissociative symptoms causing feelings or actions of the traumatic event reoccurring;
(4) intense or prolonged psychological pain upon exposure to internal or external cues that symbolize aspects of the traumatic event or something similar; and
(5) clear physiological reactions to internal or external cues that symbolize aspects of the traumatic event or something similar;

<8c> The use according to any one of <1c> to <7c>, which suppresses flashbacks; <9c> The use according to any one of <1 c> to <8c>, which suppresses avoidance of events or matters related to a trauma;
<10c> The use according to any one of <1c> to <9c>, wherein the pharmaceutical composition has a facilitation effect on the extinction of memories of a traumatic event, and/or an inhibitory effect on the reconsolidation of memories of a traumatic event;
<11c> The use according to <10c>, wherein the facilitation effect on the extinction of memories of a traumatic event

is a facilitation effect on extinction learning of memories of a traumatic event;

<12c> The use according to any one of <1c> to <11c>, wherein the pharmaceutical composition palliates fear from fear memories;

<13d> The method, use, or pharmaceutical composition according to any one of <1a> to <12a>, <1b> to <12b> and <1c> to <12c>, wherein the selective δ-opioid receptor agonist is a compound represented by the general formula (I) or a pharmaceutically acceptable salt thereof;

<14d> The method, use, or pharmaceutical composition according to any one of <1a> to <12a>, <1b> to <12b> and <1c> to <12c>, wherein the compound represented by general formula (1) disclosed in <13d> is a compound in which

R5, $R^{6a}$, $R^{6b}$, $R^7$, $R^8$, $R^9$ and $R^{10}$ all represent hydrogen,
$R^1$ represents hydrogen; $C_{1-6}$ alkyl; $C_{2-6}$ alkenyl; cycloalkylalkyl where the cycloalkyl moiety has 3 to 6 carbon atoms, and the alkylene moiety has 1 to 5 carbon atoms; or aralkyl where the aryl moiety has 6 to 10 carbon atoms, and the alkylene moiety has 1 to 5 carbon atoms,
$R^2$ represents a 5- to 7-membered heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or the heterocyclic ring condensed with a benzene ring; or pyridine 1-oxide,
$R^2$ binds to Y via a carbon atom as a ring-constituting atom of $R^2$,
$R^3$ and $R^4$, which are the same or different, represent hydrogen; hydroxy; halogen; cyano; carbamoyl; $C_{1-6}$ alkoxy; $C_{6-10}$ aryloxy; $C_{1-6}$ alkanoyloxy; amino; or acylamino where the acyl moiety has 2 to 6 carbon atoms,
X represents $CH_2$, and
Y represents C(=O),
provided that the $C_{1-6}$ alkyl as $R^1$; the alkylene moiety and cycloalkyl moiety of the cycloalkylalkyl where the cycloalkyl moiety has 3 to 6 carbon atoms, and the alkylene moiety has 1 to 5 carbon atoms as $R^1$; or the alkylene moiety of the aralkyl where the aryl moiety has 6 to 10 carbon atoms, and the alkylene moiety has 1 to 5 carbon atoms as $R^1$ may be substituted with at least one substituent selected from:

1 to 6 halogens; hydroxy; $C_{1-6}$ alkoxy; $C_{6-10}$ aryloxy; $C_{1-6}$ alkanoyl; $C_{1-6}$ alkanoyloxy; carboxyl; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; alkylsulfonyl where the alkyl moiety has 1 to 6 carbon atoms; aminosulfonyl; alkylsulfinyl where the alkyl moiety has 1 to 6 carbon atoms; alkylthio where the alkyl moiety has 1 to 6 carbon atoms; $C_{1-6}$ alkoxy substituted with 1 to 6 halogens; and arylcarbonyl where the aryl moiety has 6 to 10 carbon atoms,
the aryl moiety of the aralkyl where the aryl moiety has 6 to 10 carbon atoms, and the alkylene moiety has 1 to 5 carbon atoms as $R^1$; or the aryl moiety of the $C_{6-10}$ aryloxy as $R^3$ and $R^4$ may be substituted with at least one substituent selected from:

$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy,
the heterocyclic ring as $R^2$ may have, besides the oxo group, the substituents that the aryl moiety of the aralkyl where the aryl moiety has 6 to 10 carbon atoms, and the alkylene moiety has 1 to 5 carbon atoms as $R^1$ mentioned above may have, and pyridine 1-oxide as $R^2$ may have the substituents that the aryl moiety of the aralkyl where the aryl moiety has 6 to 10 carbon atoms, and the alkylene moiety has 1 to 5 carbon atoms as $R^1$ mentioned above may have, and
the alkylene moiety of the aralkyl where the aryl moiety has 6 to 10 carbon atoms, and the alkylene moiety has 1 to 5 carbon atoms as $R^1$ may be substituted with at least one substituent selected from phenyl, and $C_{1-6}$ alkyl substituted with 1 to 3 halogens;

<15d> The method, use, or pharmaceutical composition according to any one of <1a> to <12a>, <1b> to <12b> and <1c> to <12c>, wherein the compound represented by general formula (1) disclosed in <13d> or <14d> is a compound in which $R^1$ represents $C_{1-6}$ alkyl; cycloalkylalkyl where the cycloalkyl moiety has 3 to 6 carbon atoms, and the alkylene moiety has 1 to 5 carbon atoms; or aralkyl where the aryl moiety has 6 to 10 carbon atoms, and the alkylene moiety has 1 to 5 carbon atoms;

<16d> The method, use, or pharmaceutical composition according to any one of <1a> to <12a>, <1b> to <12b> and <1c> to <12c>, wherein the compound represented by general formula (1) disclosed in any one of <13d> to <15d> is a compound in which $R^1$ represents a cycloalkylalkyl where the cycloalkyl moiety has 3 to 6 carbon atoms, and the alkylene moiety has 1 to 5 carbon atoms;

<17d> The method, use, or pharmaceutical composition according to any one of <1a> to <12a>, <1b> to <12b> and <1c> to <12c>, wherein the compound represented by general formula (1) disclosed in <13d> or <14d> is a compound in which $R^1$ represents $C_{2-6}$ alkyl substituted with hydroxy; $C_{1-6}$ alkyl substituted with 1 to 6 halogens; or $C_{2-6}$ alkyl substituted with $C_{1-6}$ alkoxy;

<18d> The method, use, or pharmaceutical composition according to any one of <1a> to <12a>, <1b> to <12b> and <1c> to <12c>, wherein the compound represented by general formula (I) disclosed in <13d> or <14d> is a compound in which $R^1$ represents allyl, fluoropropyl, 2-(pyridin-3-yl)ethyl, 2-(methylsulfonyl)ethyl, or 2-(aminosulfonyl)ethyl;

<19d> The method, use, or pharmaceutical composition according to any one of <1a> to <12a>, <1b> to <12b> and <1c> to <12c>, wherein the compound represented by general formula (I) disclosed in any one of <13d> to <18d> is a compound in which $R^2$ represents a 5- to 7-membered heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or the heterocyclic ring condensed with a benzene ring; or pyridine 1-oxide;

<20d> The method, use, or pharmaceutical composition according to any one of <1a> to <12a>, <1b> to <12b> and <1c> to <12c>, wherein the compound represented by general formula (I) disclosed in any one of <13d> to <19d> is a compound in which $R^2$ represents pyridine 1-oxide;

<21d> The method, use, or pharmaceutical composition according to any one of <1a> to <12a>, <1b> to <12b> and <1c> to <12c>, wherein the compound represented by general formula (I) disclosed in any one of <1 3d> to <19d> is a compound in which the heterocyclic ring as $R^2$ is pyridin-2(1H)-one;

<22d> The method, use, or pharmaceutical composition according to any one of <1a> to <12a>, <1b> to <12b> and <1c> to <12c>, wherein the compound represented by general formula (1) disclosed in any one of <13d> to <19d> is a compound in which the heterocyclic ring as $R^2$ is pyridin-4(1H)-one;

<23d> The method, use, or pharmaceutical composition according to any one of <1a> to <12a>, <1b> to <12b> and <1c> to <12c>, wherein the compound represented by general formula (I) disclosed in any one of <13d> to <19d> is a compound in which the heterocyclic ring as $R^2$ is pyridazin-3(2H)-one;

<24d> The method, use, or pharmaceutical composition according to any one of <1a> to <12a>, <1b> to <12b> and <1c> to <12c>, wherein the compound represented by general formula (1) disclosed in any one of <13d> to <19d> is a compound in which the heterocyclic ring as $R^2$ is pyrazin-2(1H)-one;

<25d> The method, use, or pharmaceutical composition according to any one of <1a> to <12a>, <1b> to <12b> and <1c> to <12c>, wherein the compound represented by general formula (I) disclosed in any one of <13d> to <19d> is a compound in which the heterocyclic ring as $R^2$ is 4H-pyran-4-one or 2H-pyran-2-one;

<26d> The method, use, or pharmaceutical composition according to any one of <1a> to <12a>, <1b> to <12b> and <1c> to <12c>, wherein the compound represented by general formula (I) disclosed in any one of <1 3d> to <19d> is a compound in which the heterocyclic ring as $R^2$ is quinolin-2(1H)-one;

<27d> The method, use, or pharmaceutical composition according to any one of <1a> to <12a>, <1b> to <12b> and <1c> to <12c>, wherein the compound represented by general formula (1) disclosed in any one of <13d> to <19d> is a compound in which the heterocyclic ring as $R^2$ is pyrimidin-4(3H)-one or pyrimidin-2,4(1H,3H)-dione;

<28d> The method, use, or pharmaceutical composition according to any one of <1a> to <12a>, <1b> to <12b> and <1c> to <12c>, wherein the compound represented by general formula (I) disclosed in any one of <13d> and <15d> to <27d> is a compound in which X represents $CH_2$;

<29d> The method, use, or pharmaceutical composition according to any one of <1a> to <12a>, <1b> to <12b> and <1c> to <12c>, wherein the compound represented by general formula (I) disclosed in any one of <13d> to <28d> is a compound in which one of $R^3$ and $R^4$ represents hydroxy and the other represents hydrogen;

<30d> The method, use, or pharmaceutical composition according to any one of <1a> to <12a>, <1b> to <12b> and <1c> to <12c>, wherein the compound represented by general formula (1) disclosed in any one of <13d> and <15d> to <28d> is a compound in which $R^3$ represents halogen; cyano; carbamoyl; $C_{1-6}$ alkoxy; $C_{1-6}$ alkanoyloxy; amino; or acylamino where the acyl moiety has 2 to 6 carbon atoms; $R^4$ represents hydrogen or hydroxy; and $R^5$ represents hydrogen;

<31d> The method, use, or pharmaceutical composition according to any one of <1a> to <12a>, <1b> to <12b> and <1c> to <12c>, wherein the compound represented by general formula (I) disclosed in any one of <1 3d> and <15d> to <28d> is a compound in which $R^3$ represents hydroxy; carbamoyl; or $C_{1-6}$ alkanoyloxy; $R^4$ represents hydrogen; and $R^5$ represents hydrogen;

<32d> The method, use, or pharmaceutical composition according to any one of <1a> to <12a>, <1b> to <12b>

and <1c> to <12c>, wherein the compound represented by general formula (I) disclosed in any one of <1 3d> and <15d> to <28d> is a compound in which $R^3$ represents hydroxy; $R^4$ represents hydrogen; and $R^5$ represents hydrogen;

<33d> The method, use, or pharmaceutical composition according to any one of <1a> to <12a>, <1b> to <12b> and <1c> to <12c>, wherein the compound represented by general formula (I) disclosed in any one of <13d> and <15d> to <28d> is a compound in which $R^3$, $R^4$ and $R^5$ all represent hydrogen;

<34d> The method, use, or pharmaceutical composition according to any one of <1a> to <12a>, <1b> to <12b> and <1c> to <12c>, wherein the compound represented by general formula (I) disclosed in any one of <13d> and <15d> to <33d> is a compound in which $R^{6a}$, $R^{6b}$, $R^7$, $R^8$, $R^9$ and $R^{10}$ all represent hydrogen;

<35d> The method, use, or pharmaceutical composition according to any one of <1a> to <12a>, <1b> to <12b> and <1c> to <12c>, wherein the compound represented by general formula (I) disclosed in any one of <14d> to <28d> is a compound in which $R^3$ represents halogen; cyano; carbamoyl; $C_{1-6}$ alkoxy; $C_{1-6}$ alkanoyloxy; amino; or acylamino where the acyl moiety has 2 to 6 carbon atoms; and $R^4$ represents hydrogen or hydroxy;

<36d> The method, use, or pharmaceutical composition according to any one of <1a> to <12a>, <1b> to <12b> and <1c> to <12c>, wherein the compound represented by general formula (I) disclosed in any one of <14d> to <28d> is a compound in which $R^3$ represents hydroxy; carbamoyl; or $C_{1-6}$ alkanoyloxy; and $R^4$ represents hydrogen;

<37d> The method, use, or pharmaceutical composition according to any one of <1a> to <12a>, <1b> to <12b> and <1c> to <12c>, wherein the compound represented by general formula (1) disclosed in any one of <14d> to <28d> is a compound in which $R^3$ represents hydroxy; and $R^4$ represents hydrogen;

<38d> The method, use, or pharmaceutical composition according to any one of <1a> to <12a>, <1b> to <12b> and <1c> to <12c>, wherein the compound represented by general formula (I) disclosed in any one of <14d> to <28d> is a compound in which $R^3$ and $R^4$ both represent hydrogen;

<39d> The method, use, or pharmaceutical composition according to any one of <1a> to <12a>, <1b> to <12b> and <1c> to <12c>, wherein the compound represented by general formula (I) disclosed in <13d> is a compound represented by the following general formula (II):

[Formula 13]

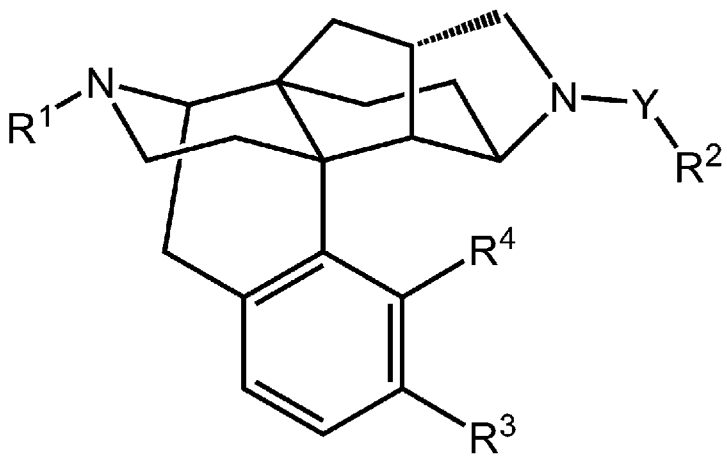

(I I)

(wherein $R^1$ represents hydrogen; $C_{1-6}$ alkyl; or cycloalkylalkyl where the cycloalkyl moiety has 3 to 6 carbon atoms, and the alkylene moiety has 1 to 5 carbon atoms;

$R^2$ represents a 5- to 7-membered heterocyclic ring containing 1 or 2 nitrogen atoms and at least one carbon atom as ring-constituting atoms, containing at least one set of adjacent ring-constituting atoms bound by a double bond, and further substituted with at least one oxo group; or pyridine 1-oxide;
$R^2$ binds to Y via a carbon atom as a ring-constituting atom of $R^2$,
$R^3$ and $R^4$, which are the same or different, represent hydrogen; hydroxy; or $C_{1-6}$ alkoxy,
Y represents C(=O),
provided that the $C_{1-6}$ alkyl as $R^1$; and the alkylene moiety and cycloalkyl moiety of the cycloalkylalkyl where the cycloalkyl moiety has 3 to 6 carbon atoms and the alkylene moiety has 1 to 5 carbon atoms as $R^1$ may be substituted with at least one substituent selected from:

1 to 6 halogens; hydroxy; and $C_{1-6}$ alkoxy,

the heterocyclic ring as $R^2$ may have, besides the oxo group, at least one substituent selected from:

$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy, and
the pyridine 1-oxide as $R^2$ may have at least one substituent selected from:
$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy);

<40d> The method, use, or pharmaceutical composition according to any one of <1a> to <12a>, <1b> to <12b> and <1c> to <12c>, wherein the compound represented by general formula (II) disclosed in <39d> is a compound in which $R^1$ is cycloalkylalkyl where the cycloalkyl moiety has 3 to 6 carbon atoms, and the alkylene moiety has 1 to 5 carbon atoms;
<41d> The method, use, or pharmaceutical composition according to any one of <1a> to <12a>, <1b> to <12b> and <1c> to <12c>, wherein the compound represented by general formula (II) disclosed in <39d> or <40d> is a compound in which $R^2$ is represented by a formula (III):

[Formula 14]

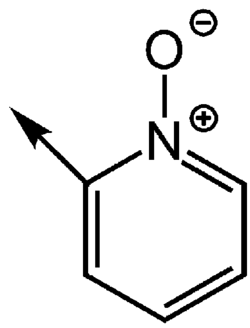

(III)

a formula (IV):

[Formula 15]

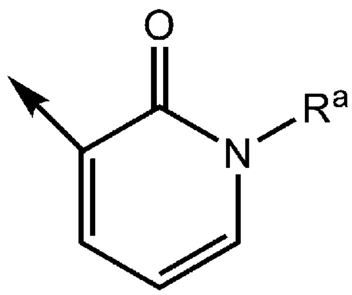

(IV)

a formula (V):

[Formula 16]

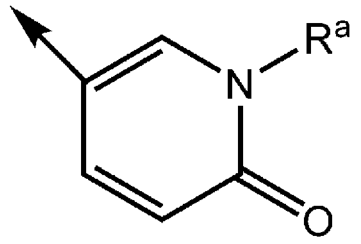

(V)

or a formula (VI):

[Formula 17]

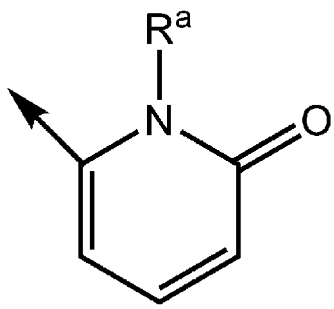

(V I)

(wherein in formulas (111) to (VI), $R^a$ represents hydrogen or $C_{1-6}$ alkyl, and binding to Y occurs at the position of the arrow);

<42d> The method, use, or pharmaceutical composition according to any one of <1a> to <12a>, <1b> to <12b> and <1c> to <12c>, wherein the compound represented by general formula (II) disclosed in any one of <39d> to <41d> is a compound in which $R^3$ is hydroxy;

<43d> The method, use, or pharmaceutical composition according to any one of <1a> to <12a>, <1b> to <12b> and <1c> to <12c>, wherein the compound represented by general formula (II) disclosed in any one of <39d> to <42d> is a compound in which $R^4$ is hydrogen;

<44d> The method, use, or pharmaceutical composition according to any one of <1a> to <12a>, <1b> to <12b> and <1c> to <12c>, wherein the compound represented by general formula (1) disclosed in <13d> is at least one compound selected from:

2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-6-methylpyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,

6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidine-2,4(1H,3H)-dione,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-4(1H)-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-4(1H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridazin-3(2H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)quinolin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-2H-pyran-2-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-4H-pyran-4-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-4(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrazin-2(1H)-one,
2-((1S,3aR,5aS,6R,11bR,11cS)-10-acetoxy-14-(cyclopropylmethyl)-2,3,3a,4,5,6,7,11c-octahydro-1H-6,11b-(epiminoethano)-1,5a-methanonaphtho[1,2-e]indole-3-carbonyl)pyridine 1-oxide,
6-((1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrazin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidine-2,4(1H,3H)-dione,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidin-4(3H)-one, and
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one;

<45d> The method, use, or pharmaceutical composition according to any one of <1a> to <12a>, <1b> to <12b> and <1c> to <12c>, wherein the compound represented by general formula (I) disclosed in any one of <13d> and <39d> to <43d> is at least one compound selected from:

2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one, and
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one.

Next, the present invention is described in further detail using a series of examples, but the present invention is not limited to these examples.

The compounds of the examples were synthesized in accordance with the method disclosed in WO2018/052114.
Nomenclature for each of the compounds of the examples was determined by converting the structural formula drawn using ChemDraw ver. 14 from CambridgeSoft to an English name using the naming algorithm included with the software, and then translating that English name into Japanese.

[Examples]

Example 1

Synthesis of 2-((1S,3aR,5aS,6R,11bR,1 1 cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridine 1-oxide

[Formula 18]

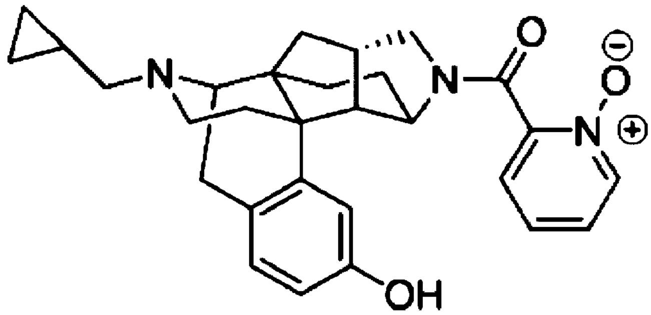

Example 2

Synthesis of 4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridine 1-oxide

[Formula 19]

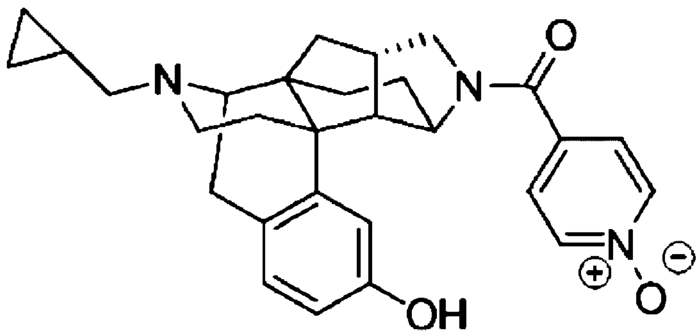

Example 3

Synthesis of 3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridin-2(1H)-one

[Formula 20]

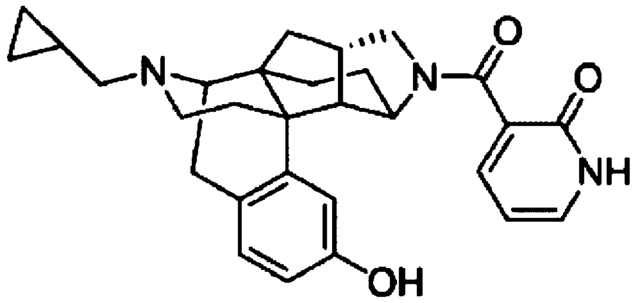

Example 4

Synthesis of 3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridine 1-oxide

[Formula 21]

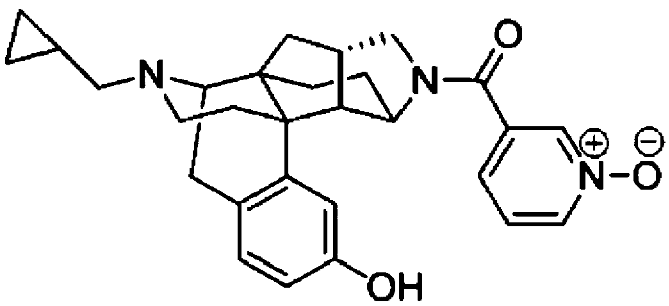

Example 5

Synthesis of 5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridin-2(1H)-one

[Formula 22]

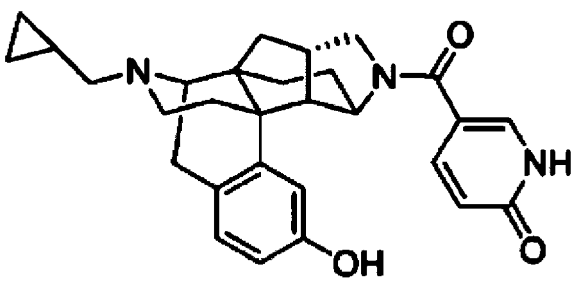

Example 6

Synthesis of 3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1-methylpyridin-2(1H)-one

[Formula 23]

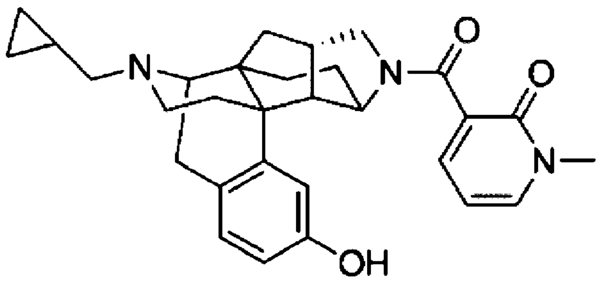

Example 7

Synthesis of 6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridin-2(1H)-one

[Formula 24]

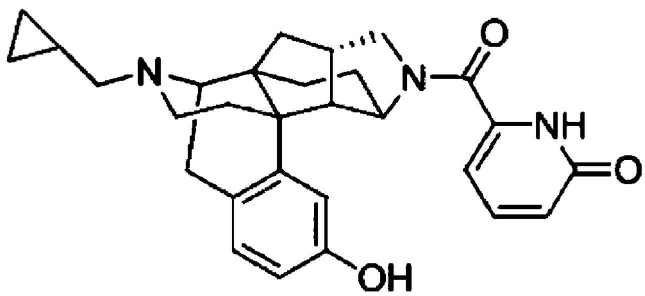

Example 8

Synthesis of 3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-6-methylpyridin-2(1H)-one

[Formula 25]

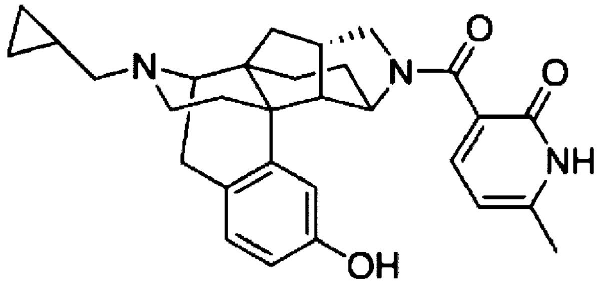

Example 9

Synthesis of 5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3 -carbonyl)-1-methylpyridin-2(1H)-one

[Formula 26]

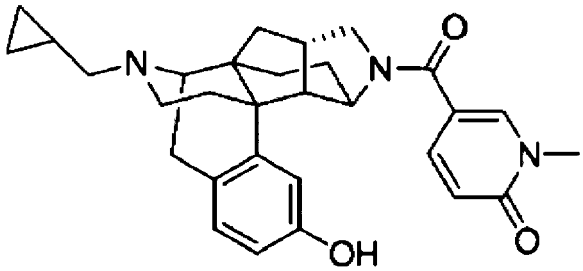

Example 10

Synthesis of 6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1-methylpyridin-2(1H)-one

[Formula 27]

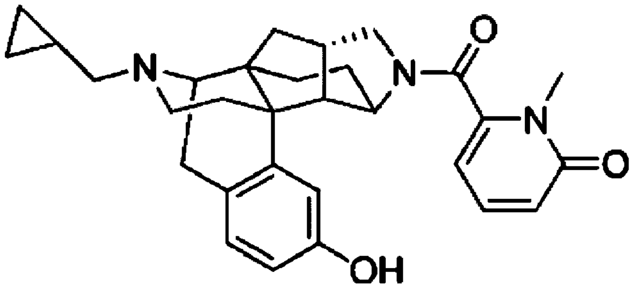

Example 11

Synthesis of 4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridin-2(1H)-one

[Formula 28]

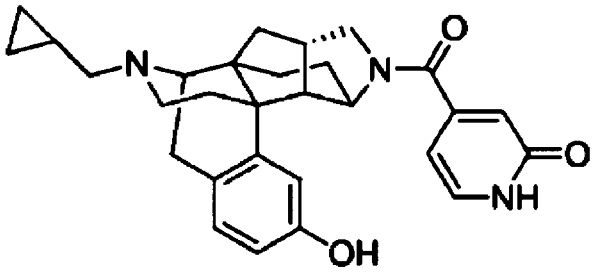

Example 12

Synthesis of 5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyrimidine-2,4(1H,3H)-dione

[Formula 29]

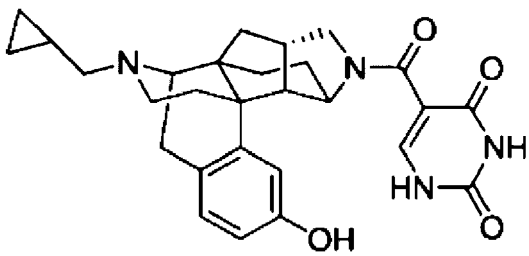

Example 13

Synthesis of 3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridin-4(1H)-one

[Formula 30]

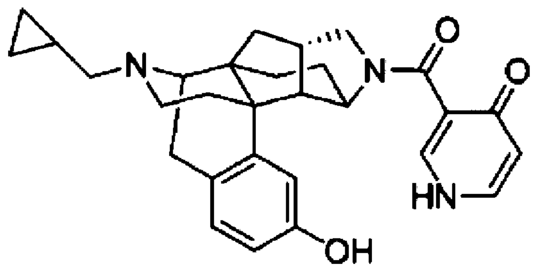

Example 14

Synthesis of 2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridin-4(1 H)-one

[Formula 31]

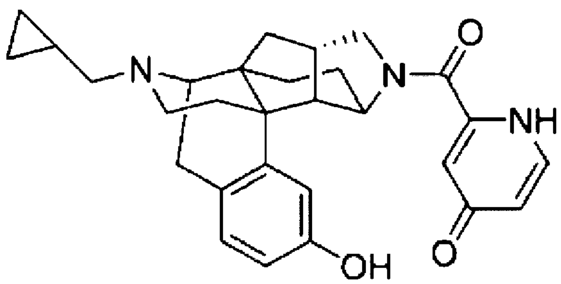

Example 15

Synthesis of 4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1-methylpyridin-2(1H)-one

[Formula 32]

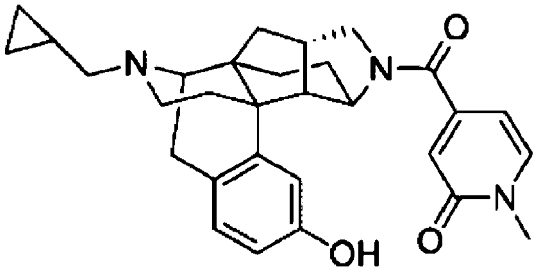

Example 16

Synthesis of 6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridazin-3(2H)-one

[Formula 33]

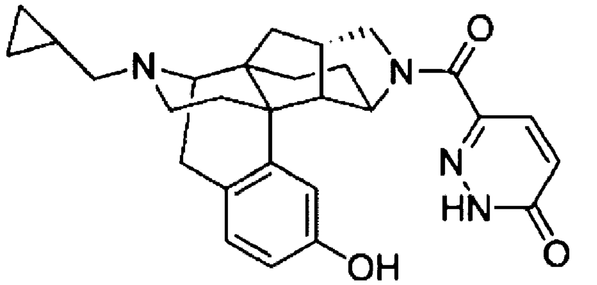

Example 17

Synthesis of 4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)quinolin-2(1H)-one

[Formula 34]

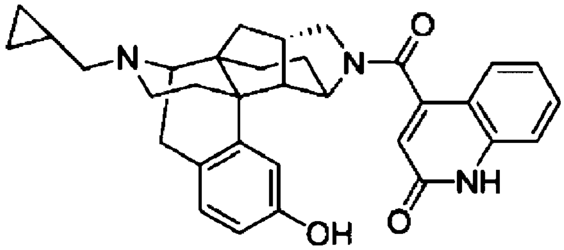

Example 18

Synthesis of 5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-2H-pyran-2-one

[Formula 35]

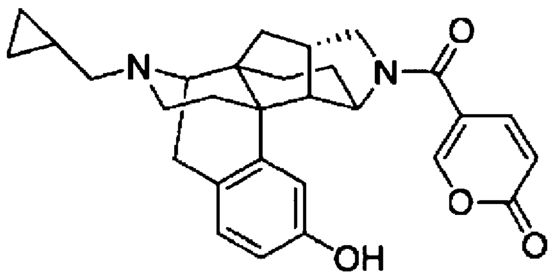

Example 19

Synthesis of 2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-4H-pyran-4-one

[Formula 36]

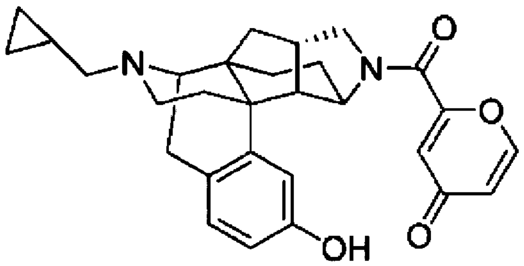

Example 20

Synthesis of 2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1-methylpyridin-4(1H)-one

[Formula 37]

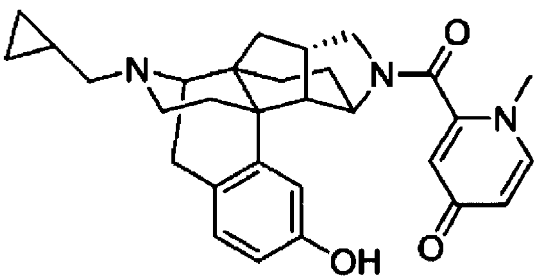

Example 21

Synthesis of 5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyrazin-2(1H)-one

[Formula 38]

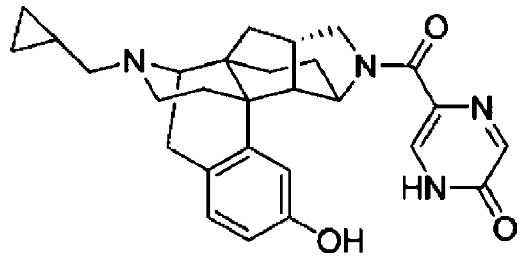

Example 22

Synthesis of 2-((1S,3aR,5aS,6R,11bR,11cS)-10-acetoxy-14-(cyclopropylmethyl)-2,3,3a,4,5,6,7,11c-octahydro-1H-6,11b-(epiminoethano)-1,5a-methanonaphtho[1,2-e] indole-3-carbonyl)pyridine 1-oxide

[Formula 39]

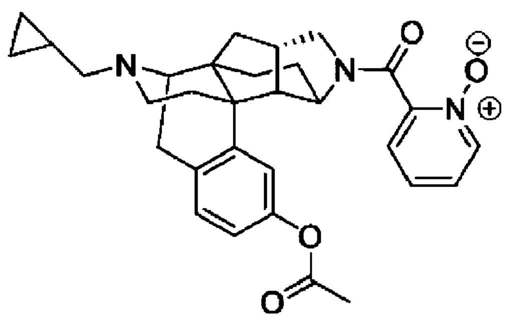

Example 23

Synthesis of 6-((1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-3-carbonyl)pyridin-2(1H)-one

[Formula 40]

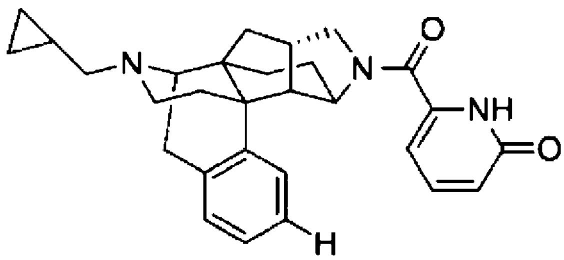

Example 24

Synthesis of 3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyrazin-2(1H)-one

[Formula 41]

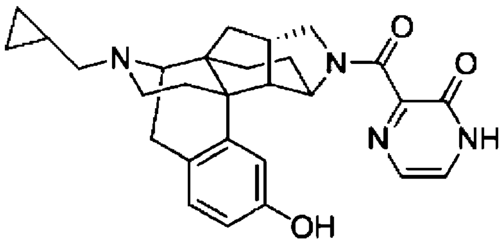

Example 25

Synthesis of 6-((1S,3aR,5aS,6R,11bR,11 cS)-14-(cyclopropylmethyl)-1 0-hydroxy-2,3,3a,4,5,6,7,11c-octahydro-1H-6,11b-(epiminoethano)-1,5a-methanonaphtho[1,2-e] indole-3-carbonyl)pyrimidine-2,4(1H,3H)-dione

[Formula 42]

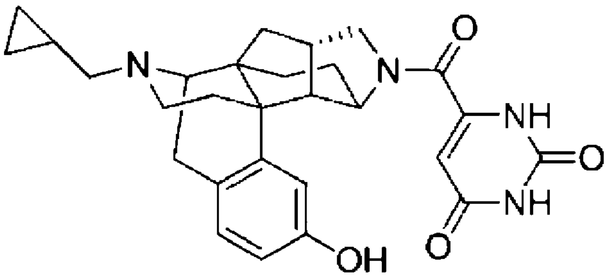

Example 26

Synthesis of 6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1 ,2-e] indole-3-carbonyl)-1-ethylpyridin-2(1H)-one

[Formula 43]

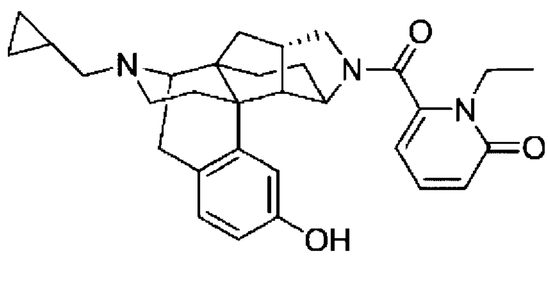

Example 27

Synthesis of 6-((1S,3aR,5aS,6R,11bR,11 cS)-14-(cyclopropylmethyl)-1 0-hydroxy-2,3,3a,4,5,6,7,11c-octahydro-1H-6,11b-(epiminoethano)-1,5a-methanonaphtho[1,2-e] indole-3-carbonyl)pyrimidin-4(3H)-one

[Formula 44]

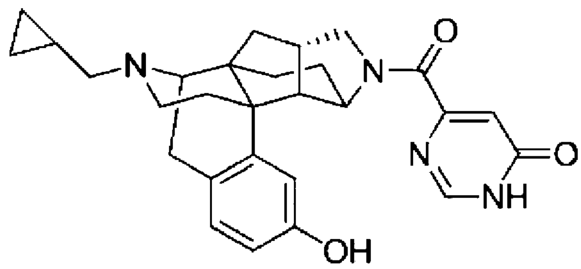

Example 28

Synthesis of 5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1-ethylpyridin-2(1H)-one

[Formula 45]

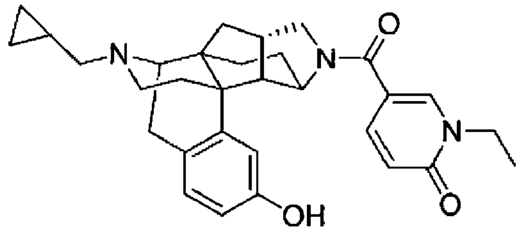

Example 29

Synthesis of 2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)pyridine 1-oxide hydrochloride

[Formula 46]

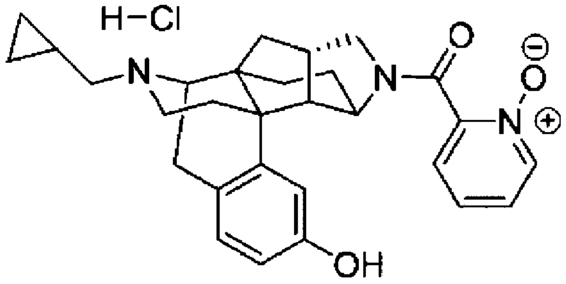

Example 30

Synthesis of 3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1 ,2-e] indole-3-carbonyl)pyridin-2(1H)-one hydrochloride

[Formula 47]

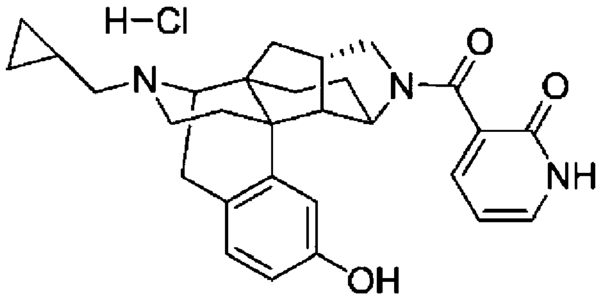

Example 31

Synthesis of 3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1-methylpyridin-2(1H)-one hydrochloride

[Formula 48]

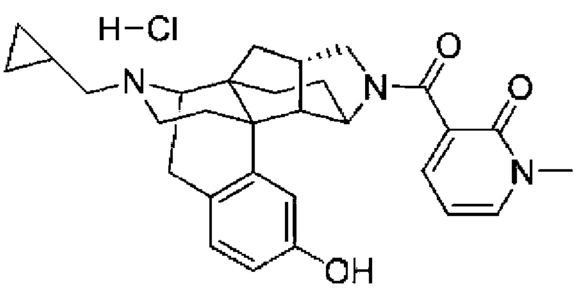

Example 32

Synthesis of 3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-6-methylpyridin-2(1H)-one hydrochloride

[Formula 49]

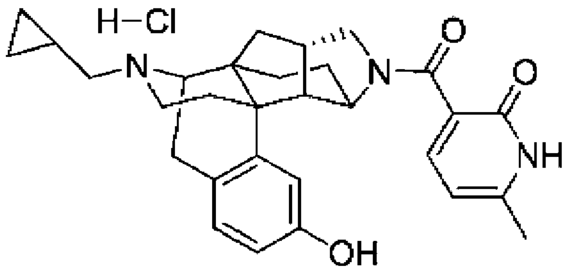

Example 33

Synthesis of 5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1-methylpyridin-2(1H)-one hydrochloride

[Formula 50]

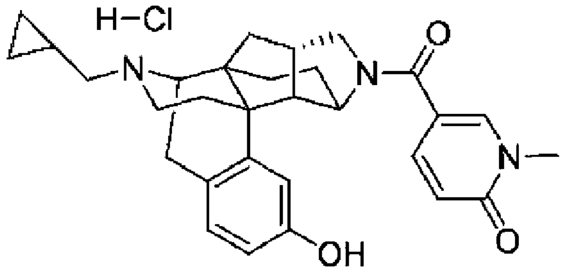

Example 34

Synthesis of 6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1-methylpyridin-2(1H)-one hydrochloride

[Formula 51]

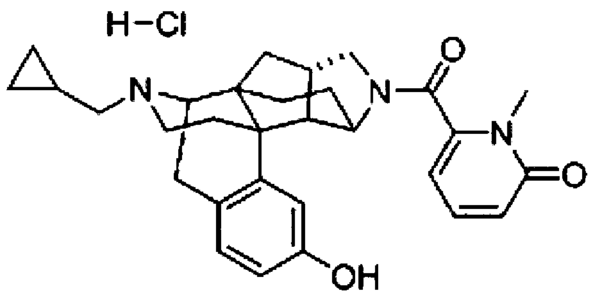

Example 35

Synthesis of 6-((1S,3aR,5aS,6R,11bR,11cS)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one

[Formula 52]

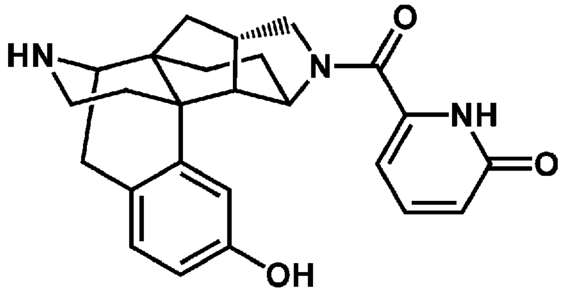

Example 36

Synthesis of 4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1-methyl-1,2-dihydro-3H-pyrazol-3-one

[Formula 53]

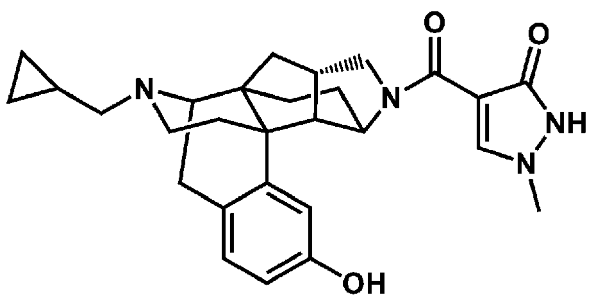

Example 37

Synthesis of 5-chloro-3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one

[Formula 54]

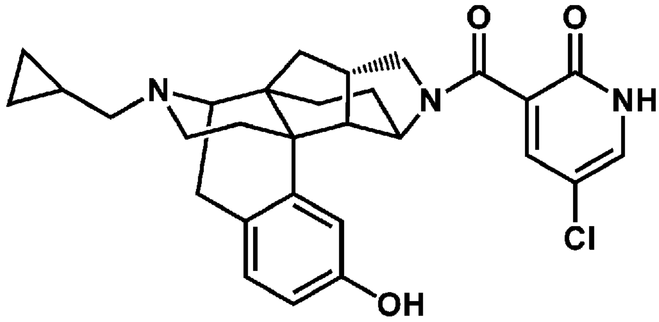

Example 38

Synthesis of 5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e] indole-3-carbonyl)-1,3-dimethylpyrimidine-2,4(1H,3H)-dione

[Formula 55]

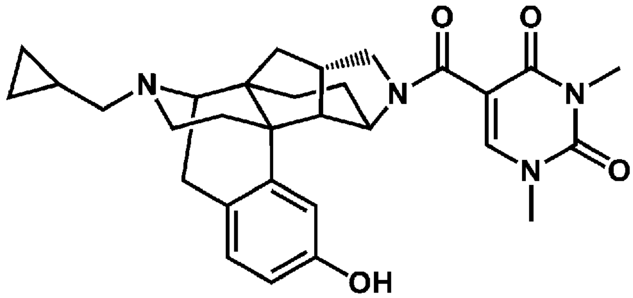

Example 39

Synthesis of 6-((1S,3aR,5aS,6R,11bR,11cS)-14-(cyclopropylmethyl)-10-methoxy-2,3,3a,4,5,6,7,11c-octahydro-1H-6,11b-(epiminoethano)-1,5a-methanonaphtho[1,2-e] indole-3-carbonyl)pyridin-2(1H)-one

[Formula 56]

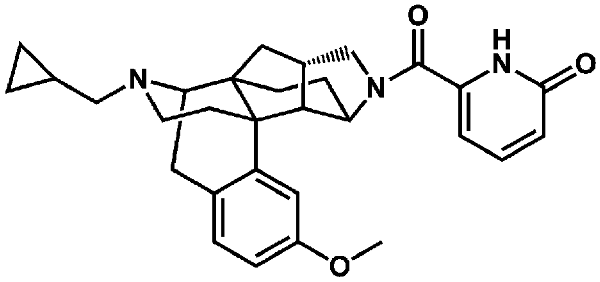

Example 40

(1) Opioid Receptor Function Test (Evaluation of Agonist Activity)

The functional activity of the compounds provided by the present invention relative to μ-, δ- and κ-opioid receptors was investigated.

Method: Using a Lance Ultra cAMP kit (manufactured by PerkinElmer, Inc.), the investigation was conducted in accordance with the prescribed method. In the evaluations of agonist activity, CHO cells expressing each of the human opioid receptors (δ, μ and κ: accession numbers and catalog numbers listed below) and each test compound were reacted for 30 minutes in an assay buffer (1×HBSS, 1 M HEPES, pH 7.4, 250 mM IBMX (isobutylmethylxanthine), 7.5% BSA) in the presence of 10 μM forskolin. Subsequently, the cAMP detection reagent included in the kit was added, and after 1 hour, a time-resolved fluorescence measurement was performed using an EnVision plate reader (manufactured by PerkinElmer, Inc.). The test compounds and each of three control drugs (δ: SNC80, μ: DAMGO, κ: U-69593) were evaluated in a concentration range from $10^{-12}$ to $10^{-5}$ M, a dose-response curve for each test compound was determined from the fluorescence value at 665 nm, and an $EC_{50}$ value and $E_{max}$ value were calculated. The $E_{max}$ value was calculated as the ratio of the maximum response of the test compound relative to a value of 100% for the maximum response of each control drug.

SNC80: (+)-4-[(αR)-α-((2S,5R)-4-allyl-2,5-dimethyl-1-piperazinyl)-3-methoxybenzyl]-N,N-diethylbenzamide
DAMGO: [D-Ala$^2$,N-MePhe$^4$,Gly-ol]enkephalin
U-69593: (+)-(5α,7α,8β)-N-methyl-N-[7-(1-pyrrolidinyl)-1-oxaspiro[4.5]dec-8-yl] benzeneacetamide
<Accession Numbers and Catalog Numbers>
δ: Catalog No. CT4607, Accession No. NM_000911.2
μ: Catalog No. CT4605, Accession No. NM_000914
κ: Catalog No. CT4606, Accession No. NM_000912

(ChanTest Corporation)

[Table 1]

| Example | δ-receptor | | μ-receptor | | κ-receptor | |
|---|---|---|---|---|---|---|
| | $EC_{50}$ value (nM) | $E_{max}$ (%) | $EC_{50}$ value (nM) | $E_{max}$ (%) | $EC_{50}$ value (nM) | $E_{max}$ (%) |
| 1 | <3 | 88 | N.C. | 8.3* | >1 | 12 |
| 3 | <3 | 98 | >1 | 20 | <1 | 15 |
| 4 | <3 | 86 | N.C. | 5.5* | >10 | 27 |
| 5 | <3 | 81 | >1 | 9.7 | <1 | 20 |
| 6 | <3 | 97 | >10 | 7.7 | N.C. | 3.2* |
| 7 | <3 | 99 | >1 | 10 | N.C. | -0.9* |
| 8 | <3 | 70 | >1 | 8.6 | <1 | 21 |
| 9 | <3 | 74 | <1 | 15 | <1 | 14 |
| 10 | <3 | 74 | N.C. | 6.0* | N.C. | -0.7* |
| 11 | <3 | 76 | >1 | 11 | N.C. | -2.5* |
| 14 | <3 | 95 | >1 | 8.1 | N.C. | 5.7* |
| 16 | <3 | 92 | >1 | 19 | <1 | 29 |
| 19 | <3 | 91 | >1 | 19 | N.C. | 0.6* |
| 28 | <3 | 92 | >1 | 16 | <1 | 13 |
| 31 | <3 | 97 | >10 | 7.7 | N.C. | 3.2* |

(continued)

| Example | δ-receptor | | μ-receptor | | κ-receptor | |
|---|---|---|---|---|---|---|
| | $EC_{50}$ value (nM) | $E_{max}$ (%) | $EC_{50}$ value (nM) | $E_{max}$ (%) | $EC_{50}$ value (nM) | $E_{max}$ (%) |
| 32 | <3 | 70 | >1 | 8.6 | <1 | 21 |
| N.C.: because a maximum response had not been reached at the maximum concentration (10 μM), the $EC_{50}$ value could not be calculated.<br>*: because a maximum response had not been reached at the maximum concentration, the response rate at the maximum concentration was recorded as a reference value. | | | | | | |

As illustrated in Table 1, it was confirmed that the compounds of the present invention have powerful agonist activity relative to the δ-opioid receptor, and either have no agonist activity or only very weak agonist activity relative to the μ- and κ-receptors.

(2) Opioid Receptor Function Test (Evaluation of Antagonist Activity)

Evaluations of antagonist activity relative to the μ- and κ-opioid receptors were conducted using the same method as (1) above. A fixed concentration of each control drug (μ: 30 nM DAMGO, κ: 100 nM U-69593), the test compound and CHO cells expressing each of the human opioid receptors were reacted for 30 minutes in an assay buffer in the presence of 10 μM forskolin. The test compounds were evaluated in a concentration range from $10^{-12}$ to $10^{-5}$ M, and subsequent operations were conducted in the same manner as the agonist activity evaluations. A dose-response curve was determined for each test compound using a value of 100% for the response of each control drug in the absence of the test compound, and the concentration that inhibited 50% of the control drug response (namely, the IC50 value) was calculated.

[Table 2]

| Example | μ-receptor $IC_{50}$ (nM) | κ-receptor $IC_{50}$ (nM) |
|---|---|---|
| 1 | 4.1 | 3.3 |
| 3 | 4.6 | 11.1 |
| 7 | 1.3 | 4.6 |
| 9 | 2.4 | 3.3 |
| 10 | 5.7 | 3.4 |
| 28 | 2.1 | 4.0 |

As illustrated in Table 2, it was confirmed that the compounds of the present invention have powerful antagonist activity relative to the μ- and κ-opioid receptors.

Example 41

<Mouse Contextual Fear Conditioning Test (Effect on Extinction Learning of Fear Memories)>

The effect of the compound of Example 7 on extinction learning of fear memories was investigated in a mouse contextual fear conditioning test. The test was conducted in accordance with the method described in Non-Patent Document 4. Administration of the compound of Example 7 (5, 10, 20 mg/kg p.o.) or a solvent was conducted two hours prior to placing the mouse in the experimental chamber on the second day. The degree of fear memories was evaluated by measuring the time for which a fear response called freezing behavior was displayed, and calculating this time as a percentage relative to the test time (6 minutes).

In this test, it was clear that for the Example 7 compound administration group, a significant reduction in fear response was observed on the second and third days compared with the solvent administration group (FIG. 1). It is known from the previous research that extinction learning occurs by re-exposure on the second day, and therefore these results indicate that the compound of Example 7 facilitates extinction learning of fear memories.

Example 42

<Mouse Contextual Fear Conditioning Test (Effect on Reconsolidation of Fear Memories)>

Using the same contextual fear conditioning test as Example 41, the effect on the reconsolidation of fear memories was investigated. The test was conducted in accordance with the method described in Non-Patent Document 4. Administration of the compound of Example 7 (20 mg/kg p.o.) or a solvent was conducted two hours prior to placing the mouse in the experimental chamber on the second day. In this test, the test time on the second and third days was set to 2 minutes.

In this test, a significant reduction in fear response was observed for the Example 7 compound administration group on the second and third days compared with the solvent administration group (FIG. 2). It is known from the previous research that reconsolidation occurs following recollection of a fear memory upon re-exposure on the second day, and therefore these results suggest that the compound of Example 7 may offer the possibility of inhibiting the reconsolidation of fear memories.

Example 43

<Mouse Contextual Fear Conditioning Test (Effect on Reconsolidation of Fear Memories)>

When the compound of Example 7 was administered subcutaneously (1 mg/kg, 3 mg/kg, s.c.) immediately after the second day test of Example 42, a significant reduction in fear response was observed for the Example 7 compound administration group on the third day compared with the solvent administration group (FIG. 3). These results suggest that the compound of Example 7 inhibits reconsolidation of fear memories.

Example 44

<Metabolic Stability Test>

(Test Method)

Human liver microsome and the test substance were reacted for a specified period of time (0 to 60 minutes), the residual amount of unchanged test substance in the reaction sample was measured, and the residual ratio was determined. Assuming an unchanged residual ratio of 100% at a reaction time of 0 hours, the residual ratio following incubation was plotted against time as a log-linear plot to determine a regression line ($y = 100e^{-kt}$, wherein k = slope of the straight line: the elimination rate constant), and the metabolic clearance CLint (mL/min/kg) was calculated using the following formula.

$$CLint^{*} = k(\text{-min}) \times 52.5\ (\text{mg MS protein/g liver}) \times 26\ (\text{g liver/kg}) \ /\ \text{MS protein (mg MS protein/mL)}$$

*: Davies, B. and Morris T., Physiological parameters in laboratory animals and humans, Pharm. Res., 10(7): 1093 to 1095, 1993.

(Test Results)

The test results are shown in Table 3.

[Table 3]

| | Example 1 | Example 7 | Example 10 | Example 16 | Comparative compound 1 |
|---|---|---|---|---|---|
| Clint | 19 | 5.6 | 13 | 18 | 25 |

Comparative compound 1: Example 93 (compound 104) of WO2013/35833.

As is evident from Table 3, the compound of the present invention has excellent metabolic stability. In contrast, it was clear that the compounds disclosed in WO2013/35833 (Patent Document 4) include compounds with poor metabolic

stability.

[Industrial Applicability]

The pharmaceutical composition of the present invention is useful for the treatment or prevention of stress-related disorders, stress-induced anxiety disorders, or stress-related disorders or anxiety disorders associated with depression.

## Claims

**1.** A pharmaceutical composition for treatment or prevention of stress-related disorders, stress-induced anxiety disorders, or stress-related disorders or anxiety disorders associated with depression, the composition comprising a selective $\delta$-opioid receptor agonist as an active component.

**2.** The pharmaceutical composition for treatment or prevention according to Claim 1, wherein the selective $\delta$-opioid receptor agonist also has a $\kappa$-opioid receptor antagonist action.

**3.** The pharmaceutical composition for treatment or prevention according to Claim 1 or 2, wherein the selective $\delta$-opioid receptor agonist also has a $\mu$-opioid receptor antagonist action and a $\kappa$-opioid receptor antagonist action.

**4.** The pharmaceutical composition for treatment or prevention according to any one of Claims 1 to 3, wherein the stress-related disorder is an acute stress disorder, post-traumatic stress disorder, or adjustment disorder.

**5.** The pharmaceutical composition for treatment or prevention according to any one of Claims 1 to 4, wherein the stress-related disorder is an acute stress disorder or post-traumatic stress disorder.

**6.** The pharmaceutical composition for treatment or prevention according to Claim 4 or 5, which functions as a pharmaceutical composition for treatment or prevention of post-traumatic stress disorder, and has an inhibitory effect on intrusion symptoms related to a traumatic event, an inhibitory effect on persistent avoidance of stimuli associated with the traumatic event, or an inhibitory effect on negative changes in cognitions and mood related to a traumatic event, that begin following the traumatic event.

**7.** The pharmaceutical composition for treatment or prevention according to Claim 6, wherein the intrusion symptoms related to a traumatic event that begin following the traumatic event include at least one symptom among (1) to (5) described below:

(1) recurrent, involuntary and intrusive painful recollections of the traumatic event;
(2) recurrent distressing dreams in which dream content and/or emotions are related to the traumatic event;
(3) dissociative symptoms causing feelings or actions of the traumatic event reoccurring;
(4) intense or prolonged psychological pain upon exposure to internal or external cues that symbolize aspects of the traumatic event or something similar; and
(5) clear physiological reactions to internal or external cues that symbolize aspects of the traumatic event or something similar.

**8.** The pharmaceutical composition for treatment or prevention according to any one of Claims 1 to 7, wherein the composition suppresses flashbacks.

**9.** The pharmaceutical composition for treatment or prevention according to any one of Claims 1 to 8, wherein the composition suppresses avoidance of events or matters related to a trauma.

**10.** The pharmaceutical composition for treatment or prevention according to any one of Claims 1 to 9, wherein the composition has a facilitation effect on extinction of memories of a traumatic event, and/or an inhibitory effect on reconsolidation of memories of a traumatic event.

**11.** The pharmaceutical composition for treatment or prevention according to Claim 10, wherein the facilitation effect on extinction of memories of a traumatic event is a facilitation effect on extinction learning of memories of a traumatic event.

**12.** The pharmaceutical composition for treatment or prevention according to any one of Claims 1 to 11, wherein the composition palliates fear from fear memories.

**13.** The pharmaceutical composition for treatment or prevention according to any one of Claims 1 to 12, wherein the selective δ-opioid receptor agonist is a compound represented by the following general formula (I):

[Formula 1]

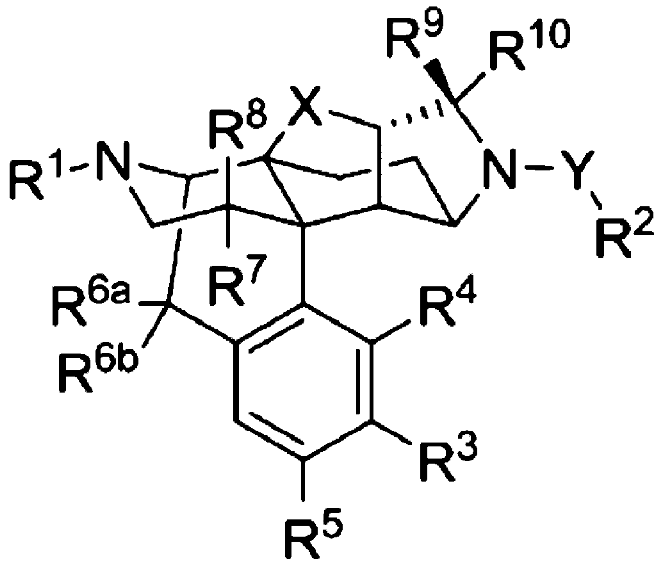

(I)

(wherein $R^1$ represents hydrogen; $C_{1-10}$ alkyl; $C_{6-10}$ aryl; $C_{2-6}$ alkenyl; cycloalkylalkyl where the cycloalkyl moiety has 3 to 6 carbon atoms, and the alkylene moiety has 1 to 5 carbon atoms; aralkyl where the aryl moiety has 6 to 10 carbon atoms, and the alkylene moiety has 1 to 5 carbon atoms; $C_{3-6}$ cycloalkyl; or heteroarylalkyl where the heteroaryl moiety contains 1 to 4 heteroatoms selected from N, O and S as ring-constituting atoms, and the alkylene moiety has 1 to 5 carbon atoms,

$R^2$ represents heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or pyridine 1-oxide,
$R^2$ binds to Y via a carbon atom as a ring-constituting atom of $R^2$,
$R^3$, $R^4$, and $R^5$, which are the same or different, represent hydrogen; hydroxy; halogen; cyano; carbamoyl; $C_{1-6}$ alkoxy; $C_{6-10}$ aryloxy; $C_{1-6}$ alkanoyloxy; nitro; amino; $C_{1-8}$ alkylamino; $C_{6-10}$ arylamino; or acylamino where the acyl moiety has 2 to 6 carbon atoms,
$R^{6a}$ and $R^{6b}$, which are the same or different, represent hydrogen; fluorine; or hydroxy, or $R^{6a}$ and $R^{6b}$ combine together to represent =O,
$R^7$ and $R^8$, which are the same or different, represent hydrogen; fluorine; or hydroxy,
$R^9$ and $R^{10}$, which are the same or different, represent hydrogen; $C_{1-6}$ alkyl; $C_{6-10}$ aryl; heteroaryl containing 1 to 4 heteroatoms selected from N, O and S as ring-constituting atoms; aralkyl where the aryl moiety has 6 to 10 carbon atoms, and the alkylene moiety has 1 to 5 carbon atoms; heteroarylalkyl where the heteroaryl moiety contains 1 to 4 heteroatoms selected from N, O and S as ring-constituting atoms, and the alkylene moiety has 1 to 5 carbon atoms; cycloalkylalkyl where the cycloalkyl moiety has 3 to 6 carbon atoms, and the alkylene moiety has 1 to 5 carbon atoms; or $C_{2-6}$ alkenyl,
X represents O or $CH_2$, and
Y represents C(=O),
provided that the $C_{1-10}$ alkyl as $R^1$; the alkylene moiety and cycloalkyl moiety of the cycloalkylalkyl where the cycloalkyl moiety has 3 to 6 carbon atoms, and the alkylene moiety has 1 to 5 carbon atoms as $R^1$; the alkylene moiety of the aralkyl where the aryl moiety has 6 to 10 carbon atoms, and the alkylene moiety has 1 to 5 carbon atoms as $R^1$; and the alkylene moiety of the heteroarylalkyl where the heteroaryl moiety contains 1 to 4 heteroatoms selected from N, O and S as ring-constituting atoms, and the alkylene moiety has 1 to 5 carbon atoms as $R^1$ may be substituted with at least one substituent selected from

1 to 6 halogens; hydroxy; $C_{1-6}$ alkoxy; $C_{6-10}$ aryloxy; $C_{1-6}$ alkanoyl; $C_{1-6}$ alkanoyloxy; carboxyl; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; alkylsulfonyl where the alkyl moiety has 1 to 6 carbon atoms; aminosulfonyl; alkylsulfinyl where the alkyl moiety has 1 to 6 carbon atoms; alkylthio where the alkyl moiety has 1 to 6 carbon atoms; $C_{1-6}$ alkoxy substituted with 1 to 6 halogens; and arylcarbonyl where the aryl moiety has 6 to 10 carbon atoms,

the $C_{6-10}$ aryl as $R^1$; the aryl moiety of the aralkyl where the aryl moiety has 6 to 10 carbon atoms, and the alkylene moiety has 1 to 5 carbon atoms as $R^1$; the aryl moiety of the $C_{6-10}$ aryloxy as $R^3$, $R^4$, or $R^5$; the aryl moiety of the $C_{6-10}$ arylamino as $R^3$, $R^4$, or $R^5$; the $C_{6-10}$ aryl as $R^9$ or $R^{10}$; the heteroaryl containing 1 to 4 heteroatoms selected from N, O and S as ring-constituting atoms as $R^9$ or $R^{10}$; the aryl moiety of the aralkyl where the aryl moiety has 6 to 10 carbon atoms, and the alkylene moiety has 1 to 5 carbon atoms as $R^9$ or $R^{10}$; and the heteroaryl moiety of the heteroarylalkyl where the heteroaryl moiety contains 1 to 4 heteroatoms selected from N, O and S as ring-constituting atoms, and the alkylene moiety has 1 to 5 carbon atoms as $R^9$ or $R^{10}$ may be substituted with at least one substituent selected from

$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy,

the heterocyclic ring as $R^2$ may have, besides the oxo group, the substituents that the $C_{6-10}$ aryl as $R^1$ mentioned above may have, and pyridine 1-oxide as $R^2$ may have the substituents that the $C_{6-10}$ aryl as $R^1$ mentioned above may have,

when $R^1$ is $C_{1-10}$ alkyl, it may be substituted with $NR^{11}R^{12}$, where $R^{11}$ and $R^{12}$, which are the same or different, represent hydrogen; $C_{1-10}$ alkyl; or aralkyl where the aryl moiety has 6 to 10 carbon atoms, and the alkylene moiety has 1 to 5 carbon atoms; or $R^{11}$, $R^{12}$, the nitrogen atom to which $R^{11}$ and $R^{12}$ bind, and optionally, 1 or 2 heteroatoms may combine together to form a 5- to 7-membered ring, and the alkylene moiety of the aralkyl where the aryl moiety has 6 to 10 carbon atoms, and the alkylene moiety has 1 to 5 carbon atoms as $R^1$ may be substituted with at least one substituent selected from phenyl, and $C_{1-6}$ alkyl substituted with 1 to 3 halogens), or a pharmaceutically acceptable salt thereof.

**14.** The pharmaceutical composition for treatment or prevention according to Claim 13, wherein the compound represented by the general formula (1) is a compound in which

$R^5$, $R^{6a}$, $R^{6b}$, $R^7$, $R^8$, $R^9$ and $R^{10}$ all represent hydrogen,

$R^1$ represents hydrogen; $C_{1-6}$ alkyl; $C_{2-6}$ alkenyl; cycloalkylalkyl where the cycloalkyl moiety has 3 to 6 carbon atoms, and the alkylene moiety has 1 to 5 carbon atoms; or aralkyl where the aryl moiety has 6 to 10 carbon atoms, and the alkylene moiety has 1 to 5 carbon atoms,

$R^2$ represents a 5- to 7-membered heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or the heterocyclic ring condensed with a benzene ring; or pyridine 1-oxide,

$R^2$ binds to Y via a carbon atom as a ring-constituting atom of $R^2$,

$R^3$ and $R^4$, which are the same or different, represent hydrogen; hydroxy; halogen; cyano; carbamoyl; $C_{1-6}$ alkoxy; $C_{6-10}$ aryloxy; $C_{1-6}$ alkanoyloxy; amino; or acylamino where the acyl moiety has 2 to 6 carbon atoms,

X represents $CH_2$, and

Y represents C(=O),

provided that the $C_{1-6}$ alkyl as $R^1$; the alkylene moiety and cycloalkyl moiety of the cycloalkylalkyl where the cycloalkyl moiety has 3 to 6 carbon atoms, and the alkylene moiety has 1 to 5 carbon atoms as $R^1$; or the alkylene moiety of the aralkyl where the aryl moiety has 6 to 10 carbon atoms, and the alkylene moiety has 1 to 5 carbon atoms as $R^1$ may be substituted with at least one substituent selected from:

1 to 6 halogens; hydroxy; $C_{1-6}$ alkoxy; $C_{6-10}$ aryloxy; $C_{1-6}$ alkanoyl; $C_{1-6}$ alkanoyloxy; carboxyl; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; alkylsulfonyl where the alkyl moiety has 1 to 6 carbon atoms; aminosulfonyl; alkylsulfinyl where the alkyl moiety has 1

to 6 carbon atoms; alkylthio where the alkyl moiety has 1 to 6 carbon atoms; $C_{1-6}$ alkoxy substituted with 1 to 6 halogens; and arylcarbonyl where the aryl moiety has 6 to 10 carbon atoms,
the aryl moiety of the aralkyl where the aryl moiety has 6 to 10 carbon atoms, and the alkylene moiety has 1 to 5 carbon atoms as $R^1$; or the aryl moiety of the $C_{6-10}$ aryloxy as $R^3$ and $R^4$ may be substituted with at least one substituent selected from:

$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy,
the heterocyclic ring as $R^2$ may have, besides the oxo group, the substituents that the aryl moiety of the aralkyl where the aryl moiety has 6 to 10 carbon atoms, and the alkylene moiety has 1 to 5 carbon atoms as $R^1$ mentioned above may have, and pyridine 1-oxide as $R^2$ may have the substituents that the aryl moiety of the aralkyl where the aryl moiety has 6 to 10 carbon atoms, and the alkylene moiety has 1 to 5 carbon atoms as $R^1$ mentioned above may have, and
the alkylene moiety of the aralkyl where the aryl moiety has 6 to 10 carbon atoms, and the alkylene moiety has 1 to 5 carbon atoms as $R^1$ may be substituted with at least one substituent selected from phenyl, and $C_{1-6}$ alkyl substituted with 1 to 3 halogens.

**15.** The pharmaceutical composition for treatment or prevention according to Claim 13 or 14, wherein the compound represented by the general formula (I) is a compound in which $R^1$ represents $C_{1-6}$ alkyl; cycloalkylalkyl where the cycloalkyl moiety has 3 to 6 carbon atoms, and the alkylene moiety has 1 to 5 carbon atoms; or aralkyl where the aryl moiety has 6 to 10 carbon atoms, and the alkylene moiety has 1 to 5 carbon atoms.

**16.** The pharmaceutical composition for treatment or prevention according to any one of Claims 13 to 15, wherein the compound represented by the general formula (I) is a compound in which $R^1$ represents a cycloalkylalkyl where the cycloalkyl moiety has 3 to 6 carbon atoms, and the alkylene moiety has 1 to 5 carbon atoms.

**17.** The pharmaceutical composition for treatment or prevention according to Claim 13 or 14, wherein the compound represented by the general formula (1) is a compound in which $R^1$ represents $C_{2-6}$ alkyl substituted with hydroxy; $C_{1-6}$ alkyl substituted with 1 to 6 halogens; or $C_{2-6}$ alkyl substituted with $C_{1-6}$ alkoxy.

**18.** The pharmaceutical composition for treatment or prevention according to Claim 13 or 14, wherein the compound represented by the general formula (I) is a compound in which $R^1$ represents allyl, fluoropropyl, 2-(pyridin-3-yl)ethyl, 2-(methylsulfonyl)ethyl, or 2-(aminosulfonyl)ethyl.

**19.** The pharmaceutical composition for treatment or prevention according to any one of Claims 13 to 18, wherein the compound represented by the general formula (I) is a compound in which $R^2$ represents a 5- to 7-membered heterocyclic ring containing 1 to 4 heteroatoms selected from N, O and S and at least one carbon atom as ring-constituting atoms, containing at least one set of adjacent ring-constituting atoms bound by a double bond, and further substituted with at least one oxo group; or the heterocyclic ring condensed with a benzene ring; or pyridine 1-oxide.

**20.** The pharmaceutical composition for treatment or prevention according to any one of Claims 13 to 19, wherein the compound represented by the general formula (I) is a compound in which $R^2$ represents pyridine 1-oxide.

**21.** The pharmaceutical composition for treatment or prevention according to any one of Claims 13 to 19, wherein the compound represented by the general formula (1) is a compound in which the heterocyclic ring as $R^2$ is pyridin-2(1H)-one.

**22.** The pharmaceutical composition for treatment or prevention according to any one of Claims 13 to 19, wherein the compound represented by the general formula (I) is a compound in which the heterocyclic ring as $R^2$ is pyridin-4(1H)-one.

**23.** The pharmaceutical composition for treatment or prevention according to any one of Claims 13 to 19, wherein the compound represented by the general formula (I) is a compound in which the heterocyclic ring as $R^2$ is pyridazin-

3(2H)-one.

**24.** The pharmaceutical composition for treatment or prevention according to any one of Claims 13 to 19, wherein the compound represented by the general formula (1) is a compound in which the heterocyclic ring as $R^2$ is pyrazin-2(1H)-one.

**25.** The pharmaceutical composition for treatment or prevention according to any one of Claims 13 to 19, wherein the compound represented by the general formula (1) is a compound in which the heterocyclic ring as $R^2$ is 4H-pyran-4-one or 2H-pyran-2-one.

**26.** The pharmaceutical composition for treatment or prevention according to any one of Claims 13 to 19, wherein the compound represented by the general formula (I) is a compound in which the heterocyclic ring as $R^2$ is quinolin-2(1H)-one.

**27.** The pharmaceutical composition for treatment or prevention according to any one of Claims 13 to 19, wherein the compound represented by the general formula (I) is a compound in which the heterocyclic ring as $R^2$ is pyrimidin-4(3H)-one or pyrimidin-2,4(1H,3H)-dione.

**28.** The pharmaceutical composition for treatment or prevention according to any one of Claims 13 and 15 to 27, wherein the compound represented by the general formula (I) is a compound in which X represents $CH_2$.

**29.** The pharmaceutical composition for treatment or prevention according to any one of Claims 13 to 28, wherein the compound represented by the general formula (I) is a compound in which one of $R^3$ and $R^4$ represents hydroxy and the other represents hydrogen.

**30.** The pharmaceutical composition for treatment or prevention according to any one of Claims 13 and 15 to 28, wherein the compound represented by the general formula (I) is a compound in which $R^3$ represents halogen; cyano; carbamoyl; $C_{1-6}$ alkoxy; $C_{1-6}$ alkanoyloxy; amino; or acylamino where the acyl moiety has 2 to 6 carbon atoms; $R^4$ represents hydrogen or hydroxy; and $R^5$ represents hydrogen.

**31.** The pharmaceutical composition for treatment or prevention according to any one of Claims 13 and 15 to 28, wherein the compound represented by the general formula (I) is a compound in which $R^3$ represents hydroxy; carbamoyl; or $C_{1-6}$ alkanoyloxy; $R^4$ represents hydrogen; and $R^5$ represents hydrogen.

**32.** The pharmaceutical composition for treatment or prevention according to any one of Claims 13 and 15 to 28, wherein the compound represented by the general formula (1) is a compound in which $R^3$ represents hydroxy; $R^4$ represents hydrogen; and $R^5$ represents hydrogen.

**33.** The pharmaceutical composition for treatment or prevention according to any one of Claims 13 and 15 to 28, wherein the compound represented by the general formula (I) is a compound in which $R^3$, $R^4$ and $R^5$ all represent hydrogen.

**34.** The pharmaceutical composition for treatment or prevention according to any one of Claims 13 and 15 to 33, wherein the compound represented by the general formula (1) is a compound in which $R^{6a}$, $R^{6b}$, $R^7$, $R^8$, $R^9$ and $R^{10}$ all represent hydrogen.

**35.** The pharmaceutical composition for treatment or prevention according to any one of Claims 14 to 28, wherein the compound represented by the general formula (1) is a compound in which $R^3$ represents halogen; cyano; carbamoyl; $C_{1-6}$ alkoxy; $C_{1-6}$ alkanoyloxy; amino; or acylamino where the acyl moiety has 2 to 6 carbon atoms; and $R^4$ represents hydrogen or hydroxy.

**36.** The pharmaceutical composition for treatment or prevention according to any one of Claims 14 to 28, wherein the compound represented by the general formula (I) is a compound in which $R^3$ represents hydroxy; carbamoyl; or $C_{1-6}$ alkanoyloxy; and $R^4$ represents hydrogen.

**37.** The pharmaceutical composition for treatment or prevention according to any one of Claims 14 to 28, wherein the compound represented by the general formula (I) is a compound in which $R^3$ represents hydroxy; and $R^4$ represents hydrogen.

**38.** The pharmaceutical composition for treatment or prevention according to any one of Claims 14 to 28, wherein the compound represented by the general formula (1) is a compound in which $R^3$ and $R^4$ both represent hydrogen.

**39.** The pharmaceutical composition for treatment or prevention according to Claim 13, wherein the compound represented by the general formula (1) is a compound represented by the following general formula (II):

[Formula 2]

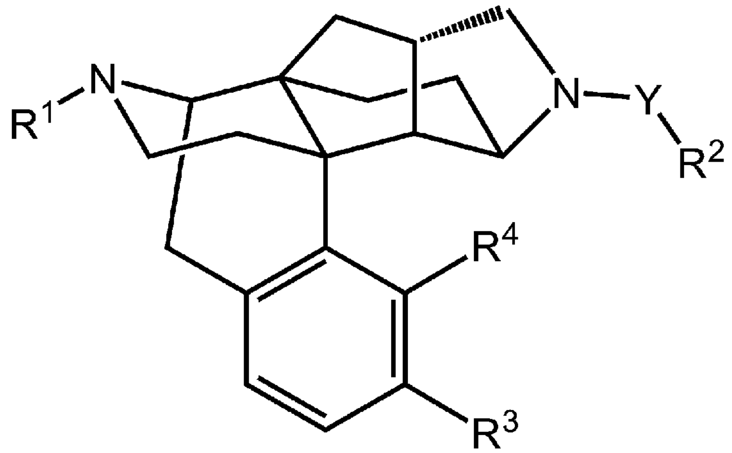

(I I)

(wherein $R^1$ represents hydrogen; $C_{1-6}$ alkyl; or cycloalkylalkyl where the cycloalkyl moiety has 3 to 6 carbon atoms, and the alkylene moiety has 1 to 5 carbon atoms;

$R^2$ represents a 5- to 7-membered heterocyclic ring containing 1 or 2 nitrogen atoms and at least one carbon atom as ring-constituting atoms, containing at least one set of adjacent ring-constituting atoms bound by a double bond, and further substituted with at least one oxo group; or pyridine 1 -oxide;
$R^2$ binds to Y via a carbon atom as a ring-constituting atom of $R^2$,
$R^3$ and $R^4$, which are the same or different, represent hydrogen; hydroxy; or $C_{1-6}$ alkoxy,
Y represents C(=O),
provided that the $C_{1-6}$ alkyl as $R^1$; and the alkylene moiety and cycloalkyl moiety of the cycloalkylalkyl where the cycloalkyl moiety has 3 to 6 carbon atoms and the alkylene moiety has 1 to 5 carbon atoms as $R^1$ may be substituted with at least one substituent selected from:

1 to 6 halogens; hydroxy; and $C_{1-6}$ alkoxy,
the heterocyclic ring as $R^2$ may have, besides the oxo group, at least one substituent selected from:

$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy, and
the pyridine 1-oxide as $R^2$ may have at least one substituent selected from:
$C_{1-6}$ alkyl; $C_{1-6}$ alkoxy; $C_{1-6}$ alkanoyloxy; hydroxy; alkoxycarbonyl where the alkoxy moiety has 1 to 6 carbon atoms; carbamoyl; alkylcarbamoyl where the alkyl moiety has 1 to 6 carbon atoms; dialkylcarbamoyl where each alkyl moiety has 1 to 6 carbon atoms; halogen; nitro; cyano; $C_{1-6}$ alkyl substituted with 1 to 3 halogens; $C_{1-6}$ alkoxy substituted with 1 to 3 halogens; phenyl; heteroaryl containing 1 to 4 heteroatoms selected from N, O and S as ring-constituting atoms; phenoxy; phenylalkyl where the alkyl has 1 to 3 carbon atoms; and methylenedioxy).

**40.** The pharmaceutical composition for treatment or prevention according to Claim 39, wherein the compound represented by the general formula (II) is a compound in which $R^1$ is cycloalkylalkyl where the cycloalkyl moiety has 3 to 6 carbon atoms, and the alkylene moiety has 1 to 5 carbon atoms.

**41.** The pharmaceutical composition for treatment or prevention according to Claim 39 or 40, wherein the compound represented by the general formula (II) is a compound in which $R^2$ is represented by a formula (III):

[Formula 3]

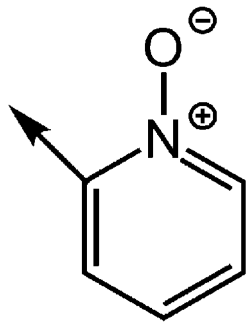

(III)

a formula (IV):

[Formula 4]

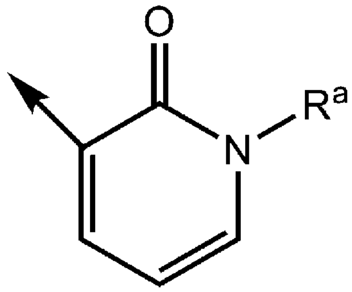

(IV)

a formula (V):

[Formula 5]

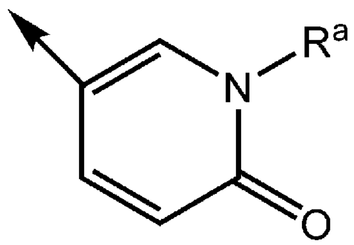

(V)

or a formula (VI):

[Formula 6]

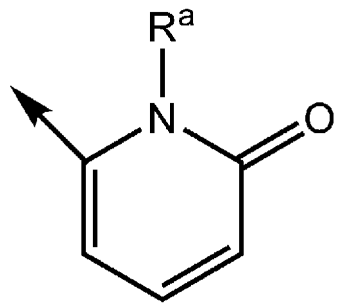

(VI)

(wherein in formulas (III) to (VI), $R^a$ represents hydrogen or $C_{1-6}$ alkyl, and binding to Y occurs at the position of the arrow).

**42.** The pharmaceutical composition for treatment or prevention according to any one of Claims 39 to 41, wherein the compound represented by the general formula (11) is a compound in which $R^3$ is hydroxy.

**43.** The pharmaceutical composition for treatment or prevention according to any one of Claims 39 to 42, wherein the compound represented by the general formula (II) is a compound in which $R^4$ is hydrogen.

**44.** The pharmaceutical composition for treatment or prevention according to Claim 13, wherein the compound represented by the general formula (1) is at least one compound selected from:

2-((1S,3aR,5aS,6R,11bR,11cS)-14-(cyclopropylmethyl)-10-hydroxy-2,3,3a,4,5,6,7,11c-octahydro-1H-6,11b-(epimioethano)-1,5a-methanonaphtho[1,2-e]indole-3-carbonyl)pyridine 1-oxide,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridine 1-oxide,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one
3-((1S,3aR,5aS,6R,11bR,11cS)-14-(cyclopropylmethyl)-10-hydroxy-2,3,3a,4,5,6,7,11c-octahydro-1H-6,11b-(epinunoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-6-methylpyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidine-2,4(1H,3H)-dione,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-4(1H)-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-4(1H)-one,
4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridazin-3(2H)-one,

4-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)quinolin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-2H-pyran-2-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-4H-pyran-4-one,
2-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-4(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrazin-2(1H)-one,
2-((1S,3aR,5aS,6R,11bR,11cS)-10-acetoxy-14-(cyclopropylmethyl)-2,3,3a,4,5,6,7,11c-octahydro-1H-6,11b-(epiminoethano)-1,5a-methanonaphtho[1,2-e]indole-3-carbonyl)pyridine 1-oxide,
6-((1S,3aR,5aS,6R,11bR,11cS)-14-(cyclopropylmethyl)-2,3,3a,4,5,6,7,11c-octahydro-1H-6,11b-(epiminoethano)-1,5a-methanonaphtho[1,2-e]indole-3-carbonyl)pyridin-2(1H)-one,
3-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrazin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidine-2,4(1H,3H)-dione,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyrimidin-4(3H)-one, and
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-ethylpyridin-2(1H)-one;

**45.** The pharmaceutical composition for treatment or prevention according to any one of Claims 13 and 39 to 43, wherein the compound represented by the general formula (I) is at least one compound selected from:

2-((1S,3aR,5aS,6R,11bR,11cS)-14-(cyclopropylmethyl)-10-hydroxy-2,3,3a,4,5,6,7,11c-octahydro-1H-6,11b-(epiuunoethano)-1,5a-methanonaphtho[1,2-e]indole-3-carbonyl)pyridine 1-oxide,
3-((1S,3aR,5aS,6R,11bR,11cS)-14-(cyclopropylmethyl)-10-hydroxy-2, 3,3a,4,5,6,7,11c-octahydro-1H-6, l lb-(epiuunoethano)-1,5a-methanonaphtho [1,2-e]indole-3-carbonyl)pyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)pyridin-2(1H)-one,
5-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one,
6-((1S,3aR,5aS,6R,11bR,11cS)-14-(cyclopropylmethyl)-10-hydroxy-2,3,3a,4,5,6,7,11c-octahydro-1H-6,11b-(epiminoethano)-1,5a-methanonaphtho[1,2-e]indole-3-carbonyl)-1-methylpyridin-2(1H)-one, and
5-((1S,3aR,5aS,6R,11bR,11cS)-14-(cyclopropylmethyl)-10-hydroxy-2,3,3a,4,5,6,7,11c-octahydro-1H-6,1b-(epiminoethano)-1,5a-methanonaphtho[1,2-e]indole-3-carbonyl)-1-ethylpyridin-2(1H)-one.

FIG. 1

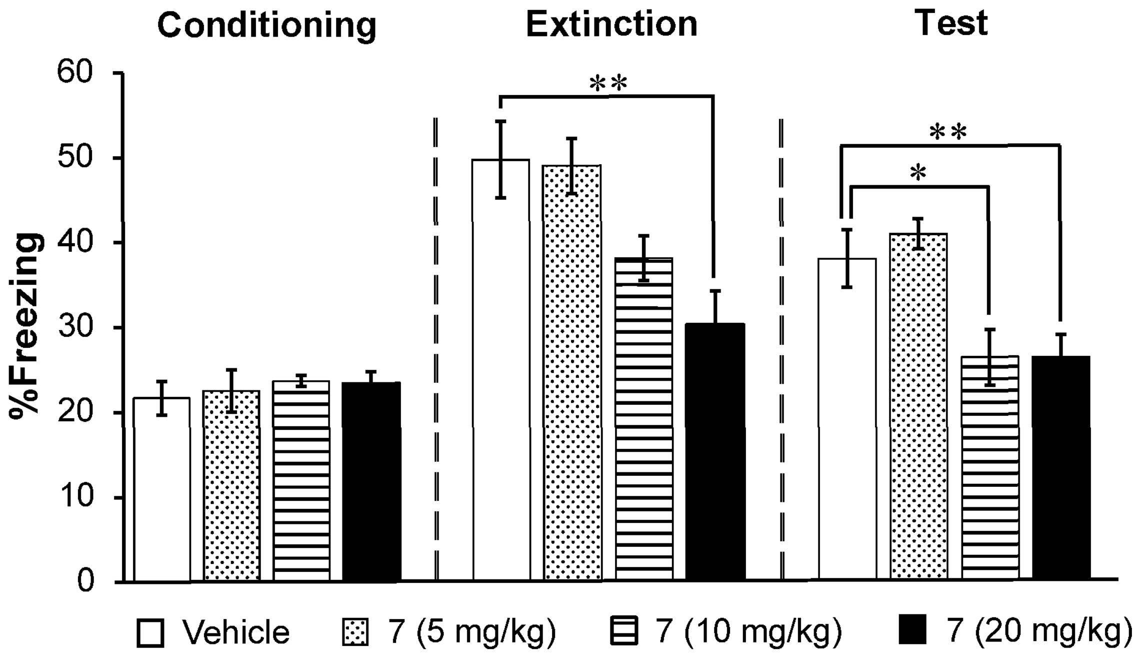


*P < 0.05, **P < 0.01 (two-way ANOVA, post hoc Bonferroni's test)

FIG. 2

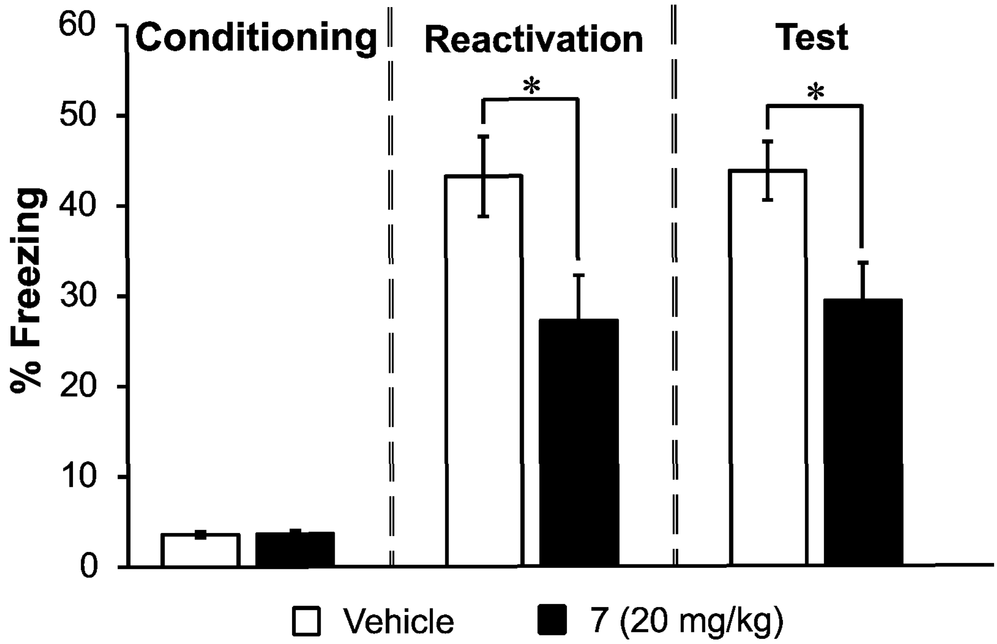


*P < 0.05 (two-way ANOVA, post hoc Bonferroni's test)

FIG. 3

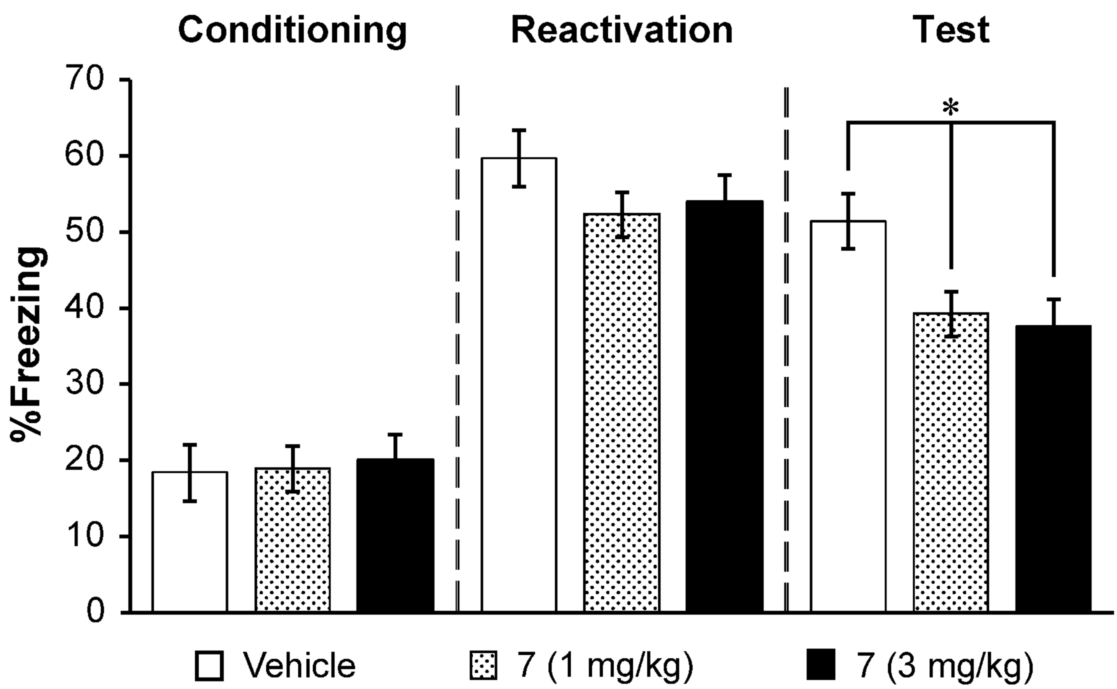


*$P < 0.05$ (two-way ANOVA, post hoc Bonferroni's test)

## INTERNATIONAL SEARCH REPORT

International application No.
**PCT/JP2022/007866**

### A. CLASSIFICATION OF SUBJECT MATTER

***A61K 45/00***(2006.01)i; ***A61P 25/00***(2006.01)i; ***A61P 25/18***(2006.01)i; ***A61P 25/22***(2006.01)i; ***A61P 25/24***(2006.01)i; ***A61P 43/00***(2006.01)i; ***A61K 31/439***(2006.01)i; ***A61K 31/444***(2006.01)i; ***A61K 31/4725***(2006.01)i; ***A61K 31/497***(2006.01)i; ***A61K 31/501***(2006.01)i; ***A61K 31/506***(2006.01)i
FI: A61K45/00; A61K31/439; A61K31/444; A61K31/506; A61K31/501; A61K31/4725; A61K31/497; A61P25/18; A61P25/22; A61P25/24; A61P25/00; A61P43/00 111

According to International Patent Classification (IPC) or to both national classification and IPC

### B. FIELDS SEARCHED

Minimum documentation searched (classification system followed by classification symbols)

A61K45/00; A61P25/00; A61P25/18; A61P25/22; A61P25/24; A61P43/00; A61K31/439; A61K31/444; A61K31/4725; A61K31/497; A61K31/501; A61K31/506

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | SUGIYAMA, A. et al. Systemic administration of a delta opioid receptor agonist, KNT-127, facilitates extinction learning of fear memory in rats. Journal of Pharmacological Sciences. 2019, vol. 139, pages 174-179, ISSN: 1347-8613<br>abstract, page 175, left column, lines 5-6 | 1, 4-12 |
| Y | abstract, page 175, left column, lines 5-6 | 1-45 |
| X | WO 2018/052114 A1 (UNIV. OF TSUKUBA) 22 March 2018 (2018-03-22)<br>claims 1-58, 62, examples 1-44, table 6, paragraph [0046] | 1-5, 13-45 |
| Y | claims 1-58, 62, examples 1-44, table 6, paragraph [0046] | 1-45 |
| A | YAMADA, D. et al. Selective agonists of the δ-opioid receptor, KNT-127 and SNC80, act differentially on extinction learning of contextual fear memory in mice. Neuropharmacology. 2019, vol. 160, #107792, ISSN: 0028-3908<br>entire text, all drawings | 1-45 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 March 2022** | **05 April 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2022/007866**

**C. DOCUMENTS CONSIDERED TO BE RELEVANT**

| Category* | Citation of document, with indication, where appropriate, of the relevant passages | Relevant to claim No. |
|---|---|---|
| A | WATANABE, Y. et al. Design and synthesis of novel delta opioid receptor agonists with an azatricyclodecane skeleton for improving blood-brain barrier penetration. Bioorganic & Medicinal Chemistry Letters. 2017, vol. 27, pages 3495-3498, ISSN: 0960-894X<br>entire text, all drawings | 1-45 |
| A | NAGASE, H. et al. Research and development of κ opioid receptor agonists and δ opioid receptor agonists. Pharmacology & Therapeutics. 2019, vol. 205, #107427, ISSN: 0163-7258<br>entire text, all drawings | 1-45 |
| A | JP 2016-95299 A (TOKYO UNIV. OF AGRICULTURE) 26 May 2016 (2016-05-26)<br>entire text, all drawings | 1-12 |
| A | 中田恵理子 他, 新規オピオイドδ受容体作動薬NC-2800の薬理作用, 鎮痛薬・オピオイドペプチドシンポジウムプログラム・抄録集, 2017, vol. 37th, page 19, non-official translation (NAKATA, Eriko et al. Pharmacological action of the novel opioid δ receptor agonist NC-2800. Analgesics/Opioid Peptide Symposium Program/Abstracts.)<br>entire text | 1-45 |

Form PCT/ISA/210 (second sheet) (January 2015)

**INTERNATIONAL SEARCH REPORT**
**Information on patent family members**

International application No.
**PCT/JP2022/007866**

| Patent document cited in search report | Publication date (day/month/year) | Patent family member(s) | Publication date (day/month/year) |
|---|---|---|---|
| WO 2018/052114 A1 | 22 March 2018 | US 2019/0328725 A1<br>claims 1-58, 62, examples 1-44, table 6, paragraphs [0255]-[0257]<br>EP 3513792 A1<br>CN 110248658 A<br>KR 10-2020-0024120 A | |
| JP 2016-95299 A | 26 May 2016 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2021030974 A
- JP 2021117338 A
- JP 2006522775 A
- WO 2001046192 A
- WO 2008001859 A
- WO 2013035833 A
- WO 2014021273 A
- WO 2014136305 A
- WO 2018052114 A
- WO 201335833 A

### Non-patent literature cited in the description

- *Tetrahedron,* 2011, vol. 67, 6682-6668
- *European Journal of Pharmacology,* 1995, vol. 276, 131-135
- *European Journal of Pharmacology,* 1997, vol. 322, 27-30
- *Neuropharmacology,* 2019, vol. 160, 107792
- *Journal of Pharmacological Sciences,* 2019, vol. 139 (3), 174-179
- *Journal of Neuroscience,* 2012, vol. 32 (27), 9335-9343
- The Diagnostic and Statistical Manual of Mental Disorders. American Psychiatric Association
- **DAVIES, B. ; MORRIS T.** Physiological parameters in laboratory animals and humans. *Pharm. Res.,* 1993, vol. 10 (7), 1093-1095